# EUROPÄISCHE PATENTSCHRIFT

(11) **EP 2 326 714 B1**
(45) Veröffentlichungstag und Bekanntmachung des Hinweises auf die Patenterteilung: **25.11.2015**
(21) Anmeldenummer: 09775637.3
(22) Anmeldetag: 18.09.2009
(51) Int. Cl.: C12N 9/10, C12N 9/18, C12N 15/52, A23K 1/165

(54) **VERFAHREN ZUR HERSTELLUNG EINES FUTTERMITTELZUSATZES ZUM SAUERSTOFFUNABHÄNGIGEN, ENZYMATISCHEN ABBAU VON MYKOTOXINEN SOWIE FUTTERMITTELZUSATZ UND VERWENDUNG DESSELBEN**
METHOD FOR THE PRODUCTION OF A FEED ADDITIVE FOR THE OXYGEN-INDEPENDENT, ENZYMATIC DECOMPOSITION OF MYCOTOXINS, FEED ADDITIVE, AND USE THEREOF
PROCÉDÉ DE PRÉPARATION D'UN ADDITIF ALIMENTAIRE POUR ANIMAUX DESTINÉ À LA DÉGRADATION ENZYMATIQUE DE MYCOTOXINES INDÉPENDANTE DE L'OXYGÈNE, ADDITIF ALIMENTAIRE POUR ANIMAUX ET UTILISATION DE CELUI-CI

(30) Priorität: 18.09.2008 AT 50208 U
(43) Veröffentlichungstag der Anmeldung: 01.06.2011
(73) Patentinhaber: Erber Aktiengesellschaft, 3130 Herzogenburg (AT)
(72) Erfinder: MOLL, Wulf-Dieter, 2000 Stockerau (AT); HARTINGER, Doris, 1200 Wien (AT); HEINL, Stefan, 1180 Wien (AT); GRIESSLER, Karin, 3140 Pottenbrunn (AT); BINDER, Eva Maria, 3430 Tulln (AT); SCHATZMAYR, Gerd, 3430 Tulln (AT); GRABHERR, Reingard, 3021 Preßbaum (AT)
(74) Vertreter: Cunow, Gerda
(86) Internationale Anmeldenummer: PCT/AT2009/000363
(87) Internationale Veröffentlichungsnummer: WO 2010/031100

(56) Entgegenhaltungen:
- WO-A1-00/04160
- WO-A2-00/04158
- WO-A2-2004/085624
- WO-A2-2006/053357
- Hartinger et al.: "Heterologous expression of genes from the fumonisin degradation gene cluster of Sphingomonas spp. MTA144 and activity of the catabolic enzymes" New Biotechnology, [Online] 13. August 2009 (2009-08-13), XP002566822 Gefunden im Internet: URL:http://dx.doi.org/10.1016/j.nbt.2009.0 6.444> [gefunden am 2010-02-04]
- Heinl S. et al.: "Identification of a fumonisin B1 degrading gene cluster in Sphingomonas spp. MTA144" New Biotechnology, [Online] 13. August 2009 (2009-08-13), XP002566823 Gefunden im Internet: URL:http://dx.doi.org/10.1016/j.nbt.2009.0 6.290> [gefunden am 2010-02-04]
- HEINL S. ET AL.: "Degradation of fumonisin B1 by the consecutive action of two bacterial enzymes" JOURNAL OF BIOTECHNOLOGY, Bd. 145, Nr. 2, 15. Januar 2010 (2010-01-15), Seiten 120-129, XP002566276

## Beschreibung

Die vorliegende Erfindung bezieht sich auf und einen Futtermittelzusatz zum sauerstoffunabhängigen, enzymatischen Abbau von Fumonisinen, in Futtermitteln bzw. im Verdauungstrakt von Tieren sowie die Verwendung desselben.

Verschiedenste Mykotoxine treten auf landwirtschaftlichen Produkten sehr häufig auf und verursachen je nach Art der Mykotoxine schwere wirtschaftliche Schäden, insbesondere in den aus den landwirtschaftlichen Produkten hergestellten Nahrungsmitteln und auch bei Tieren und Menschen, welche derartige Nahrungsmittel verzehren, wobei derartige Schäden äußerst vielfältig sind. Es wurden bereits zahlreiche Methoden entwickelt, um zu versuchen, derartige Mykotoxine zu entgiften bzw. abzubauen oder unschädlich zu machen, um die durch die Mykotoxine verursachten Schäden in den Bereichen von tierischer und menschlicher Ernährung, der Tierzucht, Viehwirtschaft, Verarbeitung von Futter und Lebensmittel und dgl. hintanzuhalten.

Unter den bekannten Mykotoxinen existiert eine Vielzahl von untereinander strukturell verwandten Mykotoxinen, wie beispielsweise die Fumonisine, von welchen Fumonisin B₁ das am häufigsten vorkommende Toxin der Gruppe ist. Jedoch sind zahlreiche Derivate und verwandte Moleküle bekannt, welche ebenfalls giftige Wirkungen bei Tieren aufweisen, wie beispielsweise im Zusammenhang mit der Fütterung von kontaminiertem Mais. Fumonisine sind in diesem Zusammenhang eine nahezu allgegenwärtige Kontamination auf Mais, welche aber auch auf anderen Getreiden, Nüssen und Gemüse einen stark negativen Effekt in Bezug auf die Tiergesundheit und Leistung darstellen.

Der mikrobielle Abbau von Fumonisinen wurde bereits in der EP 1 860 954 beschrieben, gemäß welcher Mikroorganismen zur Entgiftung von Fumonisinen und Fumonisinderivaten eingesetzt werden, bei welchen die detoxifizierenden Bakterien oder Hefen, gewählt aus genau definierten Stämmen zur Entgiftung von Fumonisinen Futtermitteln zugesetzt werden.

Auch wurden bereits katabolische Stoffwechselwege für den biologischen Abbau von Fumonisinen und die hiefür verantwortlichen Gene und Enzyme beschrieben. So beschreibt beispielsweise die EP 0 988 383 Fumonisin entgiftende Zusammensetzungen und Verfahren, wobei die eingesetzten, Fumonisin abbauenden Enzyme in erster Linie in transgenen Pflanzen produziert werden, bei welchen die Entgiftung von Fumonisinen mit Hilfe einer Aminooxidase, welche für ihre enzymatische Aktivität molekularen Sauerstoff benötigt, erfolgt.

Des Weiteren beschreibt die WO 2004/085624 Transaminasen, Deaminasen und Aminomutasen und Zusammensetzungen und Verfahren zur enzymatischen Detoxifizierung, wobei insbesondere aminierte Toxine, beispielsweise Fumonisine, entgiftet werden. In diesem Zusammenhang werden Polypeptide, welche eine Deaminaseaktivität besitzen, zur Entgiftung eingesetzt.

Aus der WO 00/04158 ist die Verwendung von Fumonisin abbauenden Aminooxidasen, bei der Herstellung Nahrungs- und Futtermitteln bzw. in der Verarbeitung von pflanzlichen Rohstoffen bekannt geworden.

Bisher bekannten Verfahren ist jedoch gemeinsam, dass sie für eine Detoxifizierung der Mykotoxine molekularen Sauerstoff für die beschriebenen, katabolischen Stoffwechselwege benötigen, wobei die insbesondere erforderlichen Aminooxidasen unter sauerstoffunabhängigen Bedingungen nicht arbeiten können. Ein Einsatz von derartigen Genen und Enzymen zur Detoxifizierung von Futtermitteln, beispielsweise im Verdauungstrakt von Tieren, ist aufgrund des im wesentlichen sauerstofffreien Milieus in dem Verdauungstrakt von Tieren nicht möglich bzw. zeigen die bekannten Gene oder Enzyme keinerlei Wirkung.

Die Erfindung zielt nun darauf ab, einen Futtermittelzusatz zur Verfügung zu stellen, mit welchem es sicher und zuverlässig gelingt, Fumonisine im Futtermittel und/oder dem Verdauungstrakt von Tieren unabhängig von Sauerstoff sicher und zuverlässig abzubauen bzw. zu detoxifizieren.

Als pflanzliche Rohstoffe werden hiebei Getreide bzw. Getreideprodukte, Gräser, Obst oder Gemüse sowie Zwischenprodukte, die diese Rohstoffe zur Herstellung von Nahrungs- und Futtermittel enthalten, wie beispielsweise Silage, Obstmaische oder dgl. verstanden.

Als Zusatzstoffe werden vor allem Futtermittelzusätze oder Nahrungsmittelzusätze verstanden.

Die für den Abbau der Fumonisine eingesetzten Nukleinsäuresequenzen bzw. die mittels dieser Nukleinsäuresequenzen in bakteriellen und eukaryotischen Wirtszellen exprimierten, in sauerstoffunabhängigem Milieu sauerstoffunabhängig katalytisch wirkenden Enzyme sind nachfolgend aufgelistet.

### Nukleinsäuren

### Sequenzen:

>Seq ID 1 (fum (Fumonisinkatabolismus) Gencluster, 15.420 bp) 
TGTCGGCGATCRGTAAACTTCTACCGTGGTCCTCGTTCGCCCACAKCATACATCACAGACRTCGGGATTTCCAACTGAAC
GGGTCCCGGCCTGCCGGCCCACATTTCCCGGAACGCCATATGGGTGATTTCGACAATCCGGTTCCAGGCGAAGATGGGTG
CGCCCCATTTAACCGCGGTCGAAAGAGGTCGATCTGGTCTTGTCCCTGAAAGGTTTTTGGCGTGCAGGGATAAACGACA
CCAAGTTGATGCTGGGACGTTATTGCGACGAAGGGAACCCCTTCGTGGCGTGCCGTCACGACTCCAGGCAGAAGGTTTGC
CGTACCGGGACCCGGATTCGTGACAATCGCGGCGACCTGTCCGGTGGTCTTGTAAATGCCCTCGGCCATATAGGCTGCGG
CGGCCTCGTGCCGCACCGGGACGAACAATATCCCATTGTCTTCGAGCGCAGCCAGGAGCGGATCCACCTCCGGCGACATG
AGGCCGAAGACATACCGGACGCCTTCGACGGCCAAACATCGTGCCAATAATTCTCCGCCCGTGAGGCGCATGACGATCTC
CAGTACGAAAGGTGAGTGCCCAGGTTCCGGCACATTCGCTGTGGTTAGTTGATGCGCTGATCGGCCAACCGACTGAGTGG
AGTTGGATGGCCGCACCTTACCCTGTCGCGCATAACTCTCAGATCCGGAAACGGACCCCGACATTAAAATAGCGGCCGAC
CGGATCATAGGCAGAGCTGGTCGGCTGGAAAAACTGCTGGGGTCGTTCGTCGCTATTGGCGGATCTCGGTCGAACAAAT
TATTGACCGATAGAAACAGCTGCTGCTTCTGGCCAAAAGCCGCGATGTCGAAGGTCAATGTCGCGTCGGTGTACCAAACC
GCCGGAGCGTGGTTCAAATTCGTATCGACGCCCTCCACATTGTCGGCATTGAACACCGATGCTGCGATGAAGCGCTGCTG
CACGAGAAGCGCCCAATCGTCGGTCGAATATCGCGCCTGGAAGTTGGCCGACCATTTTGGCGTGTCCGGTTGTCCGAGCG
AACGGATGGCGCCGAGCCGGTCGCGATGCGATAGGCAGAGGTATGGTGCGTTGCCAGCGCACGAAGACTGAACGTGCCG
CCGCCGACGGGGCGTGAGTAATAGGCCTCGAAGTCAATTCCCGCCGCTTTCTGGACAGCCAGGTTGAGATTGGGACCCGT
CACTGTGATGGTGCCGTCCGGATTCTCCGTTATGAGGTCGCAGAAGAAGGTGTTTCCTGCATCGCACGCGTCGATTTCCT
GCTGGGGAAGGAGGAAATCGATCGCGCCCTTCACCTTCACCACATAGCGATCGACCGAAAACTGAAACCCCGGCACGAAG
GCGGGGCGTAGCACCGCGCCGAATGTAAGGACGTCCGCCTTTTCAGGGCGCAAATCCGCGTTGCCGGCGGTAAAGAACCG
CGTCTGCACAGCCTGTCCGCCATAAATTGAATTGAGCGTCGCCTGACGGCCGGGGTCGAATAGCTCGACAAGGCTTGGCC
CGCGGATATCTCGCGAACGGGTCGCGCGGAACCTGAGGCCGTCGATCGGCTCATATTCTCCGCCCAGCTTCCAGGTTGTT
ACTCCACCGGACTGGCTGTAATCGGCATATCGGACGGCGCCGTTTAAGTTCAGCGAACGTCCCAGCGCGCTGTCCTTCAG
AATCGGGACGCCGATTTCGACAAAACCTTCCTTGATGTCATAGCTTCCCGAGAAGGGAAGTGGGTTGTAGAGATTGAAGC
CTCCAGGCCGACCTGCCTGCGCCGCCGGAGCCCCCCTGATTCCCGTGATCGAGGTCGTCGCCTGCGATATCGCGTCGGTT
TCCTGCCGGGCCTTCTCCTTGCGATATTCGATACCAGCGGCGACCGAGACCGGGCCCGCGCCGAACGACAGGCTATCGCC
GAGGTCGCCGGAAATCGTGAGTCCCGCCACATATTGCTCAAGCCTCAGCTGAGCGACGCCATCAGCGGTGACATAGTCGA
TGGCCGACGCGCTCGGCGAGCCTGTGCCGAAGAGATTGAGCGGCACGCAATCTTGGTCGAGGCCGGCCAGTGTTGAACGG
CAGACGATATTGCCCGCGGGATCGCGGACCGCATCGACGGCGGCGTAGAGATTGCGGTTGATGGTGAGATTGTTTTCACG
AAGCTCGAGGTCCGTAAGGCCAAAGGAGGCCGAGCCATCGAGTTTCCAGCCATTGCCAATGTCTGCCCGGAAGCCGGCAG
CGCCGCGGTAGACCTTTGCGAAATTCTCGATTTCGACCAAGGGAAAGTCGCTTGAGAAGCGACCGACAACGATCGAAGCC
TGGGCATTTCTGTCCATGAGCGTCGCGAGTGGAGCCGGAAGGAAGGCGTTATCACGGAAGATCCGGAAATTATTCGAGCC
ACCGACATGCGATATTACGAATGCACCCAGGTTGGTGTGGGAATAAGCATAGGTGCCCTCCGCATACACCTGCACAGTGT
CGGACACATCATATGCGGCGCGTAGGAACGCGTTGTAGCGAAGCTGATCCGGGGCGAAGCCGATATTCACGCGCGGTCCA
TCGCCGCCGCTCTGGAACGACGAGCTCGTAAAATTCCCGTAGTCGAAGGTCCCTAGGACTCCTCCGGGCAAAAACGCGAT
GCCTTTCAGAGGGCCGGACGTGACAAGTCCGCCGTAGGATCCGCGAGAACTGCGAATATCGGGCACGACCGTGACGCCTG
TCGTAGCGCCGGGCACGGGATATTGGCCGGCGGCGATGTCGAACCAGCGGCGACCCGTTGCTTCATCGGCCCGGATTCCG
TCCTGTCGAAAATATTCGAAGCTGCCGAGCAAGTGCAACCGGTCGTCGGCAAACGAAGTGCCGAAGGCGATCGAACCGCC
GTAGGACGGGAGGTCGCCGCGGGTTGAAACACCCGACTGGAGCTCGGCCCTGATGCCTTCCAGATCTTCGTCGAGCACGA
AGTTGATGACGCCCGAAACGGCATCGGAACCGTAGGCGGCCGAGGCGCCGCCCGTCACGACATCGACGCGCTTGACCAAC
GCCTGCGGCAGCACGTTGATATCGACCGAGCCTGTGAAATTGGTCGCGACGAAACGGTTGCCGTTCAGCAGGACGAGGTT
CCGGTTTGACCCGAGGCCGCGCATGTTGAGCAGGTTCTGACCGCTGTTCCCCGTTCCGGGTGTCGTGCCAGGTTGGAGG
TCTTCAAGCTGTCGTTGAACACGGGCAGCTGGTTGAGTGCGTCGGCAAGGTTGGTCGGAGATGCCTCCTTCAACTGCTCG
CTGGATACGGCTGTAACCGGCGTCGGCGAATTGAAGCCGTTCTGGAGGCGGCTGCCGGTCACGACGATTTCGCTCGTTCC
CCGGTCCGTGTCCGCTTCGTCCGGCTGACCTATCGATGCGGGATCGCTATCCTGAGCACTGGCAGAGACAGGAAATGCGA
GGGTGCCGAGCGCTACTGCGCCGAGCAAACTATTTGCCTTGCCGGGCTTTTCGATTCTGAACTTCCGATACATCTGCAGT
CCCTCCCGAATTGATAGGGACTCCGTTTGAGTCCCCTTGTTCTTGACGCCGCCGTCGCTCACCACGGTCCGGTCGGAGG
CTAAGCGTCGGGCCTAAGGACCCGCAATTTGAACATCAAATGCAATGATCGGAGGCTTCATTGCACTTCGCGCATAGACC
GGCGCGGTAGCTGAAAGTGCCAATAATCAGGGATTTTGCTGAACAGTTGCGGCATGACGTCCGGCATCGGCCACGCGGTT
GGCGGCATCGACGTGGCTTTCGCGTCGCCGCCCCTCAAGCACCGGCGAGTTGCATTAAAATGGGATGAGGCTGGAGAGAC
GCAAAATCTCTGAGGACCGCGCTGAACGCGCGATCCGTCGCCTCGAGGGTCTCCGTTACATCGTCAACTGTATGGGCCGC
AGAGAGAAACATATTGTGATAGGGATGAACATAGACGCCGCCCTTCAGGCACGCCGCGGCCCACGCATAGCCGATCCGAA
AATCGGGATCGTCCGCAAAGAATATTTGCGGCATCTGCGCCGGGCCCGTCTGCTTCAACTCAAGACCATGGCGCTGAGAC
TGTGCCTCCAGGCCTGCCCGCAGGGCGGCGCCGCTGGCGATCAGCGTTTCGAGATAAGGCGTCTCTCGAATGATCCTGAG
GGTTTCGATCGCGGCCGCCATCGGTACCGCAGAGAACCAGAAGGAGCCGGTCACAAATATATCCCGCGCCGCATCGCGCG
CCTTGTTCGAGCCCAGCAGGGCGGAGATCGGATAGCCATTCGCAAAGCATTTTCCCCAGCAACTGAGATCGGGTTCGATA
CCCAAATGCGTCCAGCTGCAATCGCGCGCCACCCGGAAACCTGCGCGCACATCGTCAACGACCAGAAGCGCACCGGTCTC
GTCACAACATTTTCGAGCGGTGCGCGCGAACTCAAGCTGGGCGAGGGCCTGGTCCTCAAATACTTCGTGTCGGAAAGGTG
TGGCAAAGACAGCCGCAATATCGCCATCGTGCGCCTTGAACGCGTCCGATAAGCTTTGGGCGTCGTTATAGGTATAATAT
GCGACATGCACGCGATCGGAAGCGAGAATCCCGGCAGTATGCGGAGTGTTCCACGGGGAAGCGCCATGATAGGCGCCTTT
GGCGCATAATATGGTTTTGCGCCCCGTATGGGCACGCGCGAGAACCATCGCCGTTGAGGTGGCATCGCTGCCATTTTTGC
AGAACATCGCCCAATCCGCATGACGGACCATGCCCACAAAGGCTTCGGCGAGGTTGACCATGATCTCCGAAGGACCGGTC
ATGGTGTCGCCGAGAAGTCGCTGCGCATCAGCCGCGGCTTCGATTTCGGATTGCCGGTAACCGAGCAAATTTGGCCCATA
CGCGCACATATAGTCGATATAGGGCTGCTCGTCGGCGTCCCAAATTCGTGCCCCCAGCGCGCGCCTGAAGAACTGGGGGA
ATTCTGGCGGCAGCAACCGTGTCGACTCGTGGCCGTACATCCCGCCCGGAATGACCCGTTCGGCGCGTTCTCTGAGATCT
TTCTGCCTTGTTCCGTTCGCCATAATGCACCTCTCGCGATAAATAATGGGTAAAAATCCACGAAATTCAACGATTCGTGA
TCTGAAAGAGATATATCTTGTAATATACTGTATAATTATACACAATGCGCAATCGGACGACGGATAGCGGGGCAGGGAG
GACGGGGAAATCTATGCGGAACGTCAGCGACAAGGCGCCGCCCCACGAGACGCTCACCGTAGTCGTCGCGGCAATGATCG
TTGGCACGGCCGCCTTGATGGTGCTTGGAATACAGCCCATCCTTCTCGGCGCCCTTGTAGAGGAGGGGCGTATTCCCGCC
 
GAGGGGTTGGATCGGCGGCAACGGTGGAAATACTGGCGATCGCGGCGGGAACATGCATCGGACCCGTTCTTATGAAGAC
GGGATATCTGCGGGCGAAATGCGCGGCACTCTGCTTAATGCTCGCCGCAATCAACTTCGGATTGACGTTGCCGGGTTTCG
ATTTGCCCATCGTGGCTTGCCGAGCGGCAGCGGGAGCCCTGGAAGGTCTTTCGCTCAGCGCGGCGATCCTGATCATGACT
CATAATCGGCGGCCGGACCGGCTGAGCGGAATATTTCTGGGCGCGCAGACGATACCGCAGGTAATATCTGCTTATTTGCT
CCCGACGGAGATTATTCCGCGCTGGGGAGCGCAGGCGGCTTCACGATCCTGGGCATTCTCGCGGCGATCGCCGCGATCG
CGGCTCTGTGCCTCGTCGATCGCGTTGAGCTCGATCCGACGACCGTTAACGACGACTTGCAGTGGTCACCCGCGGCGATC
GTCATTTCGATGGCGGCATTCGTTCAATTCTCGGGGGTCGGTGCCGCATGGAGCTATCTGGAGCGACTGGCTGCGCAGCA
CGGATTTTCGGGAGAAACGATCGGTATCGCCATTTCCGGGAGTTTGCTTTGCCAGGTAGGCGGGGCTTGGCTGGCCGCTT
GGATCGGTGGGCGGGTCGGATATCGCTTCGCCTTAATCGCTGGAGCCTGCTTCAGGCGGGCAACGTGATCGCATTGGCG
GTGGCCGATCAGCCAAGCTGGTTTATTTCCGCTTCCTGTGCTTTCGGCCTGTTCTGGTTGGCGATGCAGCCCTTCCAAAT
CCGCTTCGCGATCGCGATAGATAACAGCCGGCAGCTTGCTGTACTGCTGACGCCGATCGCCCTCGTCGGGTTGAGCGCGG
GGCCCTTGTTGCTCTCTCGCTTTGCCGGGGCGACCGACTTGCGCTGGATCTTTGTGGGGAGTTCGACCTTGTTGCTGGCC
AGCGCGCTTCTGTATCTTTGCGCTTCTCTGTTTCAACCGCGCGGAAAGGTGATCGCTGAAACGGTGGACGTATGAAAAAG
ACGGATCGGGGTTCGCGATGACATCGCAGGTCAAGCTTCGTAGCGCGGCAAAGCGGCCGCGCAGTCCTAAAAGCGAGCGA
GGTCTTGCTCGTTACGAGTCCTTGCTTGATGCGACCGACAGGCTGTTGGTCGATCTAGACCCCGATCAGGTCGGTCTCTA
TCAGATTGCAGAGGAAGCGGGTGCCTCACCGTCGTCCGTCTATCATTTCTTTCCGACCAAGGAAGTGGCTCATCTCGCTC
TGATGCGCCGCTATCTGGAGGGGCTCCGGAATCTCGACGCGATGGAAGTCGACATCGGCCAGCTCGAAAGCTGGCAGGAC
CTGATGAAGTTGGATCAGATCAGGGCGCGAGACTATTATAATAGCCACCCGCCCGCCCTCAAGCTTCTGTTCGGCGGATA
TGGCGGGGTCGAGGCCAGAAAGCTTGACGAGCGATACTCCGAGGAAATCGTGAGCTCCATGTATGGCAGATACAACGGCA
TTTTCCATATGCCGCAAATGGAGAATGAGGCTCTCATGTTCACGATCTGCTTCGCAATTCTCGACGCGGTATGGGCCGTC
TCCTTTCGCCGGTTCGGTGAAATTACGTCGGATTTTCTTCGGGAGGGGCAAGCGGCTTGCATTGCCTATTGCCGACACTA
TCTGCCCGAGCGAACGCCATCAGCGTGAATCCGTTCAACGATATGCAGGAATGTCCGTTGCGTTGAGTTCGGTTCTGAGT
TCGGTCGGTTAGGAGGCCCCGCGATAAACCAACGCTCTTCTGTCGAAGGGATGTCGCCTGGTTCGACCAGGCCCTGCGAA
GTCAGCCGCAATCAACGAGGCAGATGTCAACGTGGCCAGCAAGTTCAACTGTGAGTTACTCGATCTGCGATCATTTGTTG
CGGTGTATGAAACGCGAAGTTTTAGCCACGCCGCGCGGCTTCTGAATCAATCGCAGCCCGCGCTCAGCCGGAGAATCCAG
CGCCTCGAGAGTCTCGTGGGCGGTCCGTTGTTCGAGCGGACCAGTCGGTCGCTTGCCGAAACGGCGCTCGGCAAAGAGTT
GCTCCCGGTCGCCCACCGAGCGTTGGAACTTGTCGATACGTCGCTGTTTGCGTCGCCCAATGTCCGGGAGTTCCGCTGGA
CAGACATCACGATTGCCTGTGTACAGACCGCCGCCTTCCATGTTCTCCCGCGAGCTGCGCGCTTGTACATGGATCAAAAT
CCGAGGGTCCGACTCCGCATCCTTGACGTGCCGGCGGTCGAGGCTGCGGACCTGGTTGCGAGCGGCGAGGCGGAGTTCGG
CATCAGCATTGAGAGCCTGTTGCCATCAAGCCTGCGGTTCGATGCGCTCCACGAGGACCCGTTCGGCCTGGCATGCCACC
GAAGCCATCCGCTGGCGTCGCTCGAGATCCTTGAATGGACGCAATTGAAAGGTGAAAGCCTGATCGCCGTTCACCGTGCG
AGCCGGAACCGCACGTTGCTCGATGCCGAACTCGCGCGCAACAATATCGCGCTGGAATGGCGGTATGAGGTCGCGCATCT
GACGACGGCGCTGGGATTGATCGATGCGCAATTGGGTGTCGCTGTTATGCCCCGCATGGTTATGCCCCGCTCGGGTCGGT
CGGAGGTCGTCTGGCGCCCCGTCGTCGCGCCGGTCGTCCAACGCACGATCGGCATCGTTCAGCGCCGCACCGGCTCGATG
CACCCTGCCGCACAGCAATTGCTTGCGCGGCTCCGCGCGGCCTGGTCGTCCGCCAATCTGGGCGACATCGCGTCTCGCGA
AGATGGGCATCGTGACACGCGTTCTATGCGCCTGCAGCATCGATGCTCACGATCATTGCATTTGCTGAGAGACGAACGC
GAAGATACCGCTGGGTCACAGGATATCAGTCCATCGAGGCGGGAGAGAAATGTGTGAAAGAGCACCAATGCCGTGGCGGC
CGGGCGTCCCCCGCTGCGCCCGCCACGTGGCTTGCGCGGATCAGCGTTTCCCGGGGGGCCTCCGCCATCGCCTGGACCTT
CATGCTTGGCGCAACTGCCATTCCCGTGGCTGCGCAAACTGACGATCCGAAGCTCGTTCGTCATACCCAGTCGGGCGCCG
TCGAGGGCGTCGAGGGCGACGTCGAGACTTTTTTGGGAATACCCTTCGCGGCTCCGCCGGTCGGCGACCTGCGATGGCGG
CCGCCGGCTCCGCCGAGGGCGTGGCGGCACCAGGGACGGCCGCCGCTTTGCGCCCGATTGCATCGGAACGAGCGGCT
TAGAGAGGGGAGCCGGGCTGCCGGGACGAGCGAAGACTGCCTCTATCTGAATATCTGGTCTCCCAAACAGGTCGGTAAGG
GGGGGCTCCCCGTCATGATCTGGGTTTACGGCGGTGGGTTTAGCGGCGGTTCTGGCGCGGTGCCATATTATGACGGCTCT
GCGCTCGCGCAGAAGGGCGTGGTGGTCGTCACGTTCAACTATCGCGCCGGGATTCTGGGCTTTCTTGCCCATCCGGCGCT
TTCAAAGGAAAGTCCGAATGGCGTGTCGGGCAACTATGGTCTTCTCGACATGCTCGCGGCGTTCAAATGGGTTCAGAACA
ACATAAGGGAGTTCGGCGGAGACCCGAACCGTGTCACGGTCTTTGGCGAGTCCGCCGGCGCGAGCGCGCTCGGACTGCTC
CTGACCTCGCCGCTCAGTGAGAGCGCCTTCAATCAGGCGATACTGCAAAGTCCGGGTCTGGCCAGGCCGCTCGCCACGCT
TTCTGAAAGCGAAGCGAATGGGCTGGAGCTGGGAGCCGATATTTCTGCTCTACGGCGTGCCGATGCGGGCGAATTGACGA
AGATCGCGCAATCGCGAATACCCATGTCGCGCCAGTTCACCAAGCCGCGGCCGATGGGTCCGATTCTGGACGGCTATGTT
TTGCGCACCCTTGACGTCGATGCCTTCGCCAAGGGGGCCTTCCGCAAGATACCCGTTCTGGTCGGCGGAAACGCCGACGA
AGGGCGCGCTTTTACGGATCGCCTGCCGGTCAAAACGGTCCTTGAATATCGAGCCTATCTCACAGAACAATTTGGTGACG
AGGCGGACGCATGGGAGCGTTGTTATCCCGCGAACTCCGACGCCGACGTCCCCGCCGCCGTTGCCCGTCTTTTTGGGGAT
AGTCAGTTCAACAACGGGATCGAGCTGCTCTCGGCAGCCTTCGCGAAATGGCGAACGCCGCTTTGGAGATATCGCTTTAC
GGGCATTCCAGGAGCCGGCCGTCGCCCCGCCACGCATGGAGACGAAATTCCCTATGTCTTCGCAAATCTGGGGCCGTCGT
CCGTATCTATGTTTGGGTCGCTCGAAGGCGGCGCCGGGGCGTCGGACATCAAACTTGCGACCGAAATGTCCGCGGCCTGG
GTGAGCTTCGCGGTGCACGGGGTCCCCGATCAGGGCACGAAATCGCACTGGCCGCGCTTCGAGCGGCGAGGGGAGATCAT
GACTTTTGGTTCGCAGGTTGGCTCTGGGGAAGGTCTTGGAGTTTCGCCGAGCAAAGCCTGCCAACCCTCAAAATAGCGCC
CGGCCTGTGCGTGCTTCAGCACGCCGTCCCGCTTTGCGGGCGACGGGCTGTGCCCTCTGCCTAGAAGGAAGTAAGTTGCG
CTACGACGTCGCGATAATTGGAGGTGGCAACGCTGCATTGACGGCAGCCGTGACGGCGCGTGAAGCGGGGGCCTCGGTTC
TTGTGATCGAGCATGCGCCGCGCGCCATGCGCGGCGGCAACAGTCGTCACACACGCAATATGCGTACGATGCACGAACGT
CCCCTGTCGCCGTTGACCGGTGAATATTCGGCGGACGAATATTGGAATGATCTTGTCCGCGTCACGGGGGGGCGCACCGA
CGAAGAACTCGCGCGGCTCGTTATCCGCAACACCACCGACGCTATTCCCTTCATGACGCGCTGCGGTGTGCGTTTCCAGC
CCTCGCTGTCGGGCACGCTGAGTTTATCGCGAACCAACGCATTCTTCCTTGGCGGCGGGAAGGCGCTTGTAAACGCATAT
TACGCCACGGCCGAACGGCTAGGCGTCGATATTCTCTATGATTCTGAGGTGACCGAGATCAACCTTCAGCAAGGCGTCGT
GCAGCGTCTGCAATTGCGCAGCCGGGGATTCCCTGTCGAAGTGGAAGCCAAGGCTGCCATCGCCTCGTCCGGAGGATTCC
AGGCAAATCTTGACTGGCTCTCAAGCGCATGGGGGCCTGCTGCGGCGAACTTCATCGTACGGGGCACGCCATATGCGACT
GGCACGGTGCTCAAGAACCTGTTGGAGCAAGGCGTCGCCTCGGTGGGAGATCCAACCCAATGCCATGCTGTCGCGATCGA
TGGGCGAGCGCCCAAATACGACGGCGGCATCGTCACACGACTGGACTGCGTTCCCTTCTCGATCGTCGTCAACAAGGACG
CCTTGCGCTTCTACGATGAAGGCGAAGATGTGTGGCCGAAGCGTTACGCCATATGGGGTCGCTTGGTGGCACAGCAGCCT
GATCAGATCGCTTTCAGCATAATCGATCGGCAGGCCGAAGACCTCTTCATGCCGTCAGTGTTCCCCCCCGTGCAAGCGGA
CACGATCGCGGGTCTGGCCGAGAAACTCGGTCTGAATCCCGTAACCCTGGAACGCACGGTGGCCGAATTCAACGCCGCAT
GCGTGCCCGGCGAATTCGGCGGCCAAGATCTCGACGACCTCCACACCGAGGGAATCGAACCAAAGAAATCCAACTGGGCC
CGACCGATTATTGTGCCCCCGTTCAGCGCCTATCCTCTCCGGCCCGGGATCACCTTCACCTATCTCGGCGTCAAGGTAGA
CAGCCGTGCGCGGGTCATCATGGAGACAGGTGAGCCGACAAAAAACCTGTTTGCTTCGGGGGAAATAATGGCGGGCAGCA
TTCTCGGCCAAGGTTATCTCGCTGGATTTGGAATGGCGATTGGTACCGTATTCGGCCGCATCGCGGGTTGGGAGGCCGCA
 
CGTCATGCAGGATTTTGATCTCGTAAAAATGCTGTCTGACTTGCCGTCGGCGCCGGAGCTGGAAGCCAGGCGCGTTATGG
AGGTGTGCAACGCGTGCCGCTATTGCGAAGGGTTCTGCGCGGTATTTCCTGCAATGACCTTGCAGCGTCATTTCGCCAGC
GGCGATCTCAGCCACCTCGCCAATCTCTGCCACTCGTGCCAAGGTTGCTATTACGCCTGCCAATACGCCCCTCCGCATGA
GTTCGGAATAAACGTTCCAAAGGCGCTGTCGGAGTTGCGGCTCGAGAGCTACGAGCAGCATGCTTGGCCCCGGCCGGTCG
CCGCTCTCTATCGCAAGAATGCGCTCATCATTTCCATCTTGTCGGCGGCATGCATAACCGGCGTCCTTCTGCTTGCCGCC
ATCTTCAACGGGGATGCACTTTTCGCGAAACACGCATCGGTGCCCGGCGGCGGGTTTTACAACGTTATTCCTTATCAGGC
GATGATTGCCGTCGCGGCGACCACATTTCTTTATTCCGCGCTGGCGCTGGCGATCAGTCTCGTTCGCTTTTCGCGGACGA
TCGGTCTGGGAATTAAGGTTCTTTATCAGCACGTGCCGGTTCTTCGGGCGCTACGCGATGCGGCGACTCTGCGATATCTC
GGCGGCAGCGACGGCGAGGGGTGTAACGACGCGGACGAGACATTTTCGACGACCCGGCGAAAATTTCATCACGCCCTTGC
CTATGGCTTCGGACTTTGTTTCGCGGCCACAGCCACGGGCACGATCTACGATCATATGTTCGGCTGGCCGGCGCCCTATG
CGCTTTTCAGCTTGCCGGTCGTCCTAGGGACCGTTGGGGGGATCGGAATGGTCGTGGGCGCGATCGGCCTACTCTGGCTC
AAGCTGGCCGGCGAAGACGCTCCTCGATCACCGGCACTGCTTGGGCCGGATGTTGCCCTGTTGGTGCTTCTGCTTGCCAT
AGCGGCAACGGGCCTCCTCCTTTTAGCGGTCCGCAGCACCGAAGTCATGGGCGTCGCGCTCGCCGTCCATCTCGGCGTCG
TCTTGGCCTTCTTTTTGGTGATGCCATACAGCAAATTTGTCCACGGTATCTTCAGGCTCACGGCTCTCGTGCGCCATCAT
GCTGACCGCGAGGCAAGTAATGGCTTCGCCTCCAGCCCTCCCACGAAAAAGGGTTAAACAATGGAACATATGAAGTCCGT
TCGCGATCGCAGTAGCGTCATGCAGATCGTGAGAGTGGCGAGTGGCAACTGTCTCGAGCAATATGATTTCTTCGTTTACG
GCTTCTATGCGGCATATATTGCGAGAAGCTTTTTTCCGACCGGCGATAACGCGACATCGCTCATGCTTTCATTGGCCACT
TTTGGCGCTGGTTTCCTCATGAGGCCCTTGGGGGCGATTTTTCTCGGGTCCTACATCGATCGCGTCGGGCGTCGGAAAGG
CCTGATCGTGACACTCGCGATCATGGCCGTCGGAACCCTCACCATTGCGATGACTCCAAGCTATGAGGCAATTGGATTAC
TCGCACCGGTTATCGTGCTCGTCGGGCGACTTTTGCAGGGTTTTTCCGCTGGAGCAGAGTCGGGTGGCGTCTCAGTGTAC
TTGGCGGAAATTGCGTCGCCCAAATCGAGAGGCTTCTTCACCTCGTGGCAGTCTGCCAGCCAGCAGGTGGCCGTCATGAT
CGCCGCCGCGATCGGTCTTGCGCTGCAATCAACGCTTTCACCGGAGCAAATGAACGACTGGGGATGGCGGGTGCCCTTGT
TGATCGGATGCTTGATTATCCCCGTGATACTCTGGCTGCGCCGGTCTCTCCCGGAAACGAAAGCCTATCTCCACATGGAG
CACAAGGCGCATTCGATCGGCGAATCCCTCCGCGAATTGCAACAGAGCTGGGGGCTGATCTTGACGGGCATGGCGATGTC
GATCCTCACGACGACCACCTTTTACATGATTACCGCCTATACGCCGACATTTGGCGAGAAAGCACTCGGACTGAGCCCGC
AAGATGTCCTGCTGGTTACCATCATGGTCGGCGTGTCGAACTTCCTGTGGCTTCCGATCGGGGGTGCTCTCTCGGATCGT
ATCGGTAGAACCCCGATCCTACTGGTCGTGCCGGTCACCGTTCTCGCCATCGCCTTTCCCCTGATGAGCTGGCTCGTCGC
GGCACCGACATTCGGAGCGCTTGCAGCTGTTCTGCTGACTTTCTCCGCATGCTTTGGACTCTATAATGGGGCGCTCATCG
CGAGACTCACCGAGATTATGCCTCCCGCCATTAGAACCCTTGGCTTCTCGCTGGCGTTCAGTCTCGCGACCTCGCTGTTC
GGCGGCTTCACCCCATTGGTAAGTACGGCGCTAATCCACGCGACGGCAGCAATTCCGCGCCTGCAATCTGGCTCTGTTT
TGCGGCTTTCATCAGCTTCGTCGGTGTGGCCGCATCGACCCGGCTGAGCCGGCCAATCGCCGAAGGCGCCAGATAGGACA
ATCAGAGAATGCCCGTGCGGCAATGAAGCGAGATTCGGGCGGTAGGTGCGCTGGCGGCACTTCGCGAAGAGCCGTTGCGG
ACGGCTGAAACGATGATGGTATGAATGGGCTAAGACATGAGAGCAGTAGTTTACCGAAATGGCGAACTTGTCCTGGGGGC
CTATGCTGATCCGATACCCGCCGCCGGGCAGGTGCTCGTCAAGACCAGAGCATGCGGCATCTGCGGATCTGACCTTCATT
TTTGCGATCATGCGCAGGCGTTTACGAACCTTGCATCGCGGGCGGGTATCGCCTCTATGGAAGTTGATTTGTGTCGAGAC
ATCGTTCTGGGGCATGAATTCTGTGGCGAGATTATGGAGTTCGGGCCCTCTGCGGATCGTCGCTTCAAACCCGGACAGCT
TGTGTGCTCGCTGCCGCTGGCGATCGGTCCGACCGGAGCGCGGACGATTGGCTACTCGGATGAGTATCCCGGCGGGCTCG
GCGAATATATGGTCCTCACGGAAGCGCTCTTGCTGCCTGTTCCGAACGGCCTTCCGGCGACCTGCGCGGCGTTGACGGAG
CCGATGGCGGTGGGATGGCATGCCGTCGAGATCGCGCAGGTTCAACCACATCACATCCCTGTGGTGATCGGGTGCGGACC
GGTCGGGTTGGCAGTCGTCGCTGCCCTGAAACATAAGCAAGTTGCTCCGATTATTGCGTCGGATCCATCGCCCGATCGGC
GTGCTCTTGCTCTGCGGATGGGCGCCGACGCCGTTGTCGATCCGCGCGAAGAATCACCCTTTCGCCAGGCCGAGAAGATC
GCACGCCCGGTCGGACAAGGTGGGGCCCTGTCCAGCTCATTGCTGTCAAAGTCTCAAATGATATTCGAATGCGTAGGGGT
GCCGGGCATGCTTCGGCATGCGATGGACGGCGCGTCCGACGGTCCGAGATCATGGTCGTTGGCGCATGCATGCAGCCGG
ACGCGATCGAGCCCATGATCGGGATGTTTAAAGCGCTCACGATCAAATTCTCGCGAACTTACACGGGTGAGGAATTCGCC
GCGGTGCTTCACATGATAGGTGAGGGCGCACTCGACGTATCTCCGCTCGTTACCGATGTGATTGGCCTGTCCGATGTCCC
GTCCGCGTTTGAGGCTCTACGGAGTCCAGGCGCCCAAGCAAAAGTGATTGTGGACCCTTGGCGCTGAGCCTGAGGATGCC
AAGGGTGCGACGTTGGGCATCGTCAAAGAAGGCGACGTTGACCCGGTATGTGAACATCCCCATATTCTTCCGCAGCTGAA
GCAGTTGGTAAACATGCCAAAATATGAACTGTAGTATTGCGTCGGGGTTCTCATTGTGGGGTTTGCCATTGTCATCGCTC
GCACCCGGCGACAAAGATTAGATGTACTTCCGATAATCCGTGCTCTCGACCTGGCCTTCCTTCATATATTTCAGGACCTC
TCCGACCATGCGTGCGGCGCGGATCGGGATCGGCAGGCGTTGGTTCATCTGGGTCGAGTTCCAGTTGATCTTCGTAAGAG
AGAACACCTCCTCGGCTAACTGCGCCGCGGTACTATCGCAGGATCGTCTCGAGCGTYCGC
>Seq ID 8 (*fumD*) 
GTGAAAGAGCACCAATGCCGTGGCGGCCGGGCGTCCCCCGCTGCGCCCGCCACGTGGCTTGCGCGGATCAGCGTTTCCCG
GGGGGCCTCCGCCATCGCCTGGACCTTCATGCTTGGCGCAACTGCCATTCCCGTGGCTGCGCAAACTGACGATCCGAAGC
TCGTTCGTCATACCCAGTCGGGCGCCGTCGAGGGCGTCGAGGGCGACGTCGAGACTTTTTTGGGAATACCCTTCGCGGCT
CCGCCGGTCGGCGACCTGCGATGGCGGCCGCCGGCTCCGCCGAGGGCGTGGGCGGGCACCAGGACGGCCGCCGCTTTGC
GCCCGATTGCATCGGGAACGAGCGGCTTAGAGAGGGGAGCCGGGCTGCCGGGACGAGCGAAGACTGCCTCTATCTGAATA
TCTGGTCTCCCAAACAGGTCGGTAAGGGGGGGCTCCCCGTCATGATCTGGGTTTACGGCGGTGGGTTTAGCGGCGGTTCT
GGCGCGGTGCCATATTATGACGGCTCTGCGCTCGCGCAGAAGGGCGTGGTGGTCGTCACGTTCAACTATCGCGCCGGGAT
TCTGGGCTTTCTTGCCCATCCGGCGCTTTCAAAGGAAAGTCCGAATGGCGTGTCGGGCAACTATGGTCTTCTCGACATGC
TCGCGGCGTTCAAATGGGTTCAGAACAACATAAGGGAGTTCGGCGGAGACCCGAACCGTGTCACGGTCTTTGGCGAGTCC
GCCGGCGCGAGCGCGCTCGGACTGCTCCTGACCTCGCCGCTCAGTGAGAGCGCCTTCAATCAGGCGATACTGCAAAGTCC
GGGTCTGGCCAGGCCGCTCGCCACGCTTTCTGAAAGCGAAGCGAATGGGCTGGAGCTGGGAGCCGATATTTCTGCTCTAC
GGCGTGCCGATGCGGGCGAATTGACGAAGATCGCGCAATCGCGAATACCCATGTCGCGCCAGTTCACCAAGCCGCGGCCG
ATGGGTCCGATTCTGGACGGCTATGTTTTGCGCACCCTTGACGTCGATGCCTTCGCCAAGGGGGCCTTCCGCAAGATACC
CGTTCTGGTCGGCGGAAACGCCGACGAAGGGCGCGCTTTTACGGATCGCCTGCCGGTCAAAACGGTCCTTGAATATCGAG
CCTATCTCACAGAACAATTTGGTGACGAGGCGGACGCATGGGAGCGTTGTTATCCCGCGAACTCCGACGCCGACGTCCCC
GCCGCCGTTGCCCGTCTTTTTGGGGATAGTCAGTTCAACAACGGGATCGAGCTGCTCTCGGCAGCCTTCGCGAAATGGCG
AACGCCGCTTTGGAGATATCGCTTTACGGGCATTCCAGGAGCCGGCCGTCGCCCCGCCACGCATGGAGACGAAATTCCCT
ATGTCTTCGCAAATCTGGGGCCGTCGTCCGTATCTATGTTTGGGTCGCTCGAAGGCGGCGCCGGGGCGTCGGACATCAAA
CTTGCGACCGAAATGTCCGCGGCCTGGGTGAGCTTCGCGGTGCACGGGGTCCCCGATCAGGGCACGAAATCGCACTGGCC
GCGCTTCGAGCGGCGAGGGGAGATCATGACTTTTGGTTCGCAGGTTGGCTCTGGGGAAGGTCTTGGAGTTTCGCCGAGCA
AAGCCTGCCAACCCTCAAAATAG
 >Seq ID 18 (*fumI*)

ATGGCGAACGGAACAAGGCAGAAAGATCTCAGAGAACGCGCCGAACGGGTCATTCCGGCGGGATGTACGGCCACGAGTCG
ACACGGTTGCTGCCGCCAGAATTCCCCCAGTTCTTCAGGCGCGCGCTGGGGGCACGAATTTGGGACGCCGACGAGCAGCCC
TATATCGACTATATGTGCGCGTATGGGCCAAATTTGCTCGGTTACCGGCAATCCGAAATCGAAGCCGCGGCTGATGCGCAG
CGACTTCTCGGCGACACCATGACCGGTCCTTCGGAGATCATGGTCAACCTCGCCGAAGCCTTTGTGGGCATGGTCCGTCAT
GCGGATTGGGCGATGTTCTGCAAAAATGGCAGCGATGCCACCTCAACGGCGATGGTTCTCGCGCGTGCCCATACGGGGCGC
AAAACCATATTATGCGCCAAAGGCGCCTATCATGGCGCTTCCCCGTGGAACACTCCGCATACTGCCGGGATTCTCGCTTCC
GATCGCGTGCATGTCGCATATTATACCTATAACGACGCCCAAAGCTTATCGGACGCGTTCAAGGCGCACGATGGCGATATT
GCGGCTGTCTTTGCCACACCTTTCCGACACGAAGTATTTGAGGACCAGGCCCTCGCCCAGCTTGAGTTCGCGCGCACCGCT
CGAAAATGTTGTGACGAGACCGGTGCGCTTCTGGTCGTTGACGATGTGCGCGCAGGTTTCCGGGTGGCGCGCGATTGCAGC
TGGACGCATTTGGGTATCGAACCCGATCTCAGTTGCTGGGGAAAATGCTTTGCGAATGGCTATCCGATCTCCGCCCTGCTG
GGCTCGAACAAGGCGCGCGATGCGGCGCGGGATATATTTGTGACCGGCTCCTTCTGGTTCTCTGCGGTACCGATGGCGGCC
GCGATCGAAACCCTCAGGATCATTCGAGAGACGCCTTATCTCGAAACGCTGATCGCCAGCGGCGCCGCCCTGCGGGCAGGC
CTGGAGGCACAGTCTCAGCGCCATGGTCTTGAGTTGAAGCAGACGGGCCCGGCGCAGATGCCGCAAATATTCTTTGCGGAC
GATCCCGATTTTCGGATCGGCTATGCGTGGGCCGCGGCGTGCCTGAAGGGCGGCGTCTATGTTCATCCCTATCACAATATG
TTTCTCTCTGCGGCCCATACAGTTGACGATGTAACGGAGACCCTCGAGGCGACGGATCGCGCGTTCAGCGCGGTCCTCAGA
GATTTTGCGTCTCTCCAGCCTCATCCCATTTTAATGCAACTCGCCGGTGCTTGA

### Enzyme

### Sequenzen:

>Seq ID 9 (FumD) 
VKEHQCRGGRASPAAPATWLARISVSRGASAIAWTFMLGATAIPVAAQTDDPKLVRHTQS
GAVEGVEGDVETFLGIPFAAPPVGDLRWRPPAPPRAWAGTRDGRRFAPDCIGNERLREGS
RAAGTSEDCLYLNIWSPKQVGKGGLPVMIWVYGGGFSGGSGAVPYYDGSALAQKGVVVVT
FNYRAGILGFLAHPALSKESPNGVSGNYGLLDMLAAFKWVQNNIREFGGDPNRVTVFGES
AGASALGLLLTSPLSESAFNQAILQSPGLARPLATLSESEANGLELGADISALRRADAGE
LTKIAQSRIPMSRQFTKPRPMGPILDGYVLRTLDVDAFAKGAFRKIPVLVGGNADEGRAF
TDRLPVKTVLEYRAYLTEQFGDEADAWERCYPANSDADVPAAVARLFGDSQFNNGIELLS
AAFAKWRTPLWRYRFTGIPGAGRRPATHGDEIPYVFANLGPSSVSMFGSLEGGAGASDIK
LATEMSAAWVSFAVHGVPDQGTKSHWPRFERRGEIMTFGSQVGSGEGLGVSPSKACQPSK
>Seq ID 19 (FumI)

MANGTRQKDLRERAERVIPGGMYGHESTRLLPPEFPQFFRRALGARIWDADEQPYIDYMC
AYGPNLLGYRQSEIEAAADAQRLLGDTMTGPSEIMVNLAEAFVGMVRHADWAMFCKNGSD
ATSTAMVLARAHTGRKTILCAKGAYHGASPWNTPHTAGILASDRVHVAYYTYNDAQSLSD
AFKAHDGDIAAVFATPFRHEVFEDQALAQLEFARTARKCCDETGALLVVDDVRAGFRVAR
DCSWTHLGIEPDLSCWGKCFANGYPISALLGSNKARDAARDIFVTGSFWFSAVPMAAAIE
TLRIIRETPYLETLIASGAALRAGLEAQSQRHGLELKQTGPAQMPQIFFADDPDFRIGYA
WAAACLKGGVYVHPYHNMFLSAAHTVDDVTETLEATDRAFSAVLRDFASLQPHPILMQLA
GA

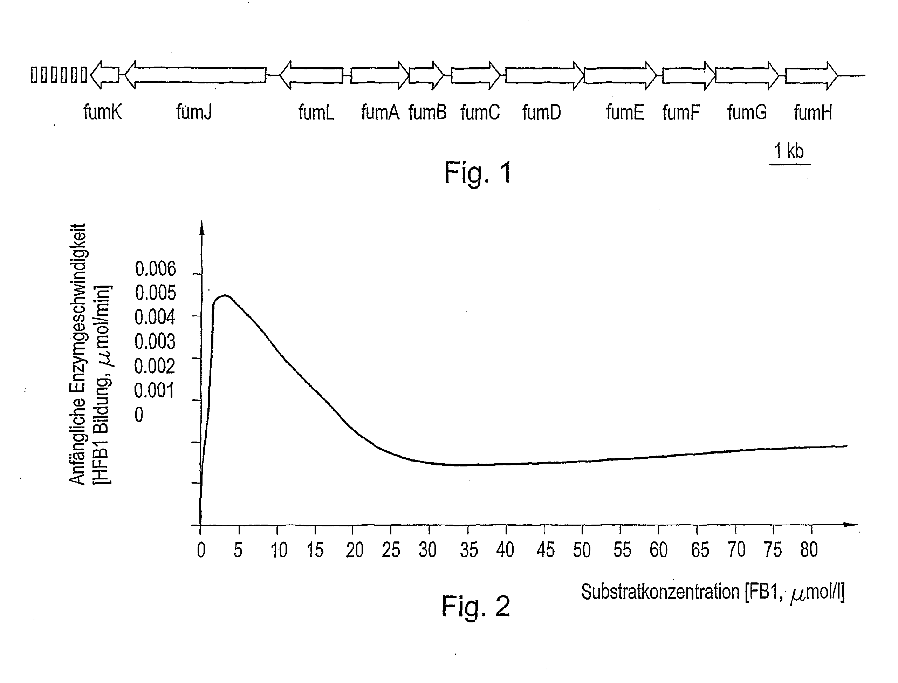

Indem hierbei das Enzym eine Carboxylesterase Sequenz ID-Nr. 9 ist, können insbesondere Fumonisine vollständig und sauerstoffunabhängig abgebaut werden. In diesem Fall werden aus dem Gencluster der Nukleinsäuresequenz mit der ID-Nr. 1 ausgewählte offene Leserahmen in prokaryotischen oder eukaryotischen Wirtszellen zur Expression gebracht. Im bakteriellen Stamm mit der Hinterlegungsnummer DSM 16254 erfolgt die Transkription der im Gencluster mit der Sequenz ID-Nr. 1 enthaltenen offenen Leserahmen gesteuert bzw. geregelt durch einen bidirektionalen Promotor, der zwischen fumA und fumI, wie dies Abb. 1 entnehmbar ist, angeordnet ist. Die Gene codieren für Proteine, welche in der Regulierung der Genexpression, wie beispielsweise FumB und FumC, bei der Substraterkennung, dem Transport und im Substratkatabolismus, wie beispielsweise FumD oder FumI involviert sind. Aus den entsprechenden Nukleinsäuresequenzen, welche für spezielle Proteine codieren, wurden gemäß einer bevorzugten Weiterbildung des Verfahrens gemäß der Erfindung die Gene ausgewählt, welche für den Substratkatabolismus verantwortlich sind, wodurch die entsprechenden gebildeten Enzyme das Substrat, nämlich Fumonisine vollständig katabolisieren können.

In der nachfolgenden Tabelle 1 ist die Bezeichnung der Gene des fumonisinkatabolischen Genclusters aufgeführt, wobei O die Orientierung, nämlich f forward und r reverse bedeuten.

**Tabelle 1**

| Gen | Sequenz ID | O | Start | Ende | Länge | Bezeichnung |
|---|---|---|---|---|---|---|
| *fumD* | 8 | f | 8294 | 9916 | 1623 | Carboxylesterase |
| *fumI* | 18 | r | 5063 | 3795 | 1269 | Aminotransferase |

Zur Lösung dieser Aufgabe ist ein derartiger Futtermittelzusatz im wesentlichen dadurch gekennzeichnet, dass das Enzym der Sequenz ID-Nr. 9 und 1 sowie gegebenenfalls zusätzlich wenigstens ein Enzym der Sequenz ID-Nr. 19, ein Cosubstrat für wenigstens eines bzw. einige der eingesetzten Enzyme und ein inerter Träger enthalten sind. Ein derartiger Futtermittelzusatz, in dem wenigstens ein Enzym sowie gegebenenfalls zusätzlich wenigstens ein Cosubstrat für wenigstens eines der eingesetzten Enzyme und ein inerter Träger enthalten sind, zeichnet sich dadurch aus, dass er zielgerichtet Mykotoxine, insbesondere Fumonisine im Verdauungstrakt von Tieren abbaut und somit detoxifiziert. Durch den Einsatz eines erfindungsgemäßen Futtermittelzusatzes, welcher im wesentlichen aus isolierten Enzymen sowie gegebenenfalls deren Cosubstraten und Trägern bestehen, ergibt sich der Vorteil, dass diese ihre katalytische Aktivität in einer Umgebung und unter Bedingungen beibehalten, in welchem beispielsweise komplette Mikroorganismen nicht oder nur wenig aktiv wären, wobei gleichzeitig eine bedeutend höhere, spezifische Aktivität erzielt werden kann, sowie definierte Reaktionen unter Vermeidung von unerwünschten Nebenreaktionen katalysiert werden können. Darüber hinaus können Probleme, welche gemäß dem Stand der Technik durch den Einsatz vermehrungsfähiger Keime in den Futtermitteln entstanden sind, mit Sicherheit hintangehalten werden und überdies weisen Futtermittelzusätze, welche nur isolierte Enzyme enthalten, sowohl eine bessere Eignung zur Formulierung für gezielte und kontrollierte Aktivierung, d.h. beispielsweise an einer bestimmten Stelle des Verdauungstrakts, als auch die Vermeidung von unerwünschtem, erhöhtem Substratverbrauch auf. Um die Spezifität noch weiter zu erhöhen, kann der Futtermittelzusatz gemäß der vorliegenden Erfindung dahingehend weitergebildet sein, dass durch molekulargenetische Methoden, Mutagenese oder molekulare Evolution veränderte Enzyme eingesetzt sind.

Mit einem derartigen Zusatzstoff gelingt es weiterhin, den Sphingolipidstoffwechsel, der durch eine Wechselwirkung der Fumonisine mit dem Enzym Ceramidsynthase behindert wird, aufrecht zu erhalten und gleichzeitig die Fumonisine biologisch zu nicht toxischen Substanzen abzubauen, welche von dem Tier ohne Schädigung ausgeschieden werden können.

Schließlich können technologische Anwendungen der Detoxifizierung erzielt werden, da dieser Futtermittelzusatz auch im größeren technischen Maßstab anwendbar ist, so dass sicher und zuverlässig die Fumonisine im sauerstoffunabhängigen Milieu enzymatisch abgebaut werden können.

Indem der Futtermittelzusatz so ausgebildet ist, wie dies einer bevorzugten Weiterbildung der Erfindung entspricht, dass das Enzym mit einer Schutzhülle ummantelt eingesetzt ist, kann sichergestellt werden, dass das Enzym vor einem vorzeitigen Aktivitätsverlust gesichert ist und sicher und zuverlässig an der vorgesehenen Stelle des Magen-Darm-Trakts ihre Wirkung entfaltet.

Durch Verkapseln des Enzyms in einer Schutzhülle gelingt es, das Enzym ohne Veränderung, insbesondere ohne Abbau und Schädigung an ihren Einsatzort, insbesondere in den Verdauungstrakt von Tieren, zu transportieren, so dass erst nach Auflösung der Schutzhülle, beispielsweise im Magen-Darm-Trakt von Tieren, das Enzym zu wirken beginnt, wodurch ein noch gezielterer, rascherer und vollständiger Abbau der Mykotoxine im sauerstoffunabhängigen bzw. anaeroben Milieu des Magen-Darm-Trakts von Tieren erzielt werden kann.

Indem der Futtermittelzusatz so ausgebildet ist, dass das Enzym eine Carboxylesterase Sequenz ID-Nr. 9 ist, wird im wesentlichen das zum Substratkatabolismus befähigte Enzym zum Einsatz gebracht, so dass neben einer geringeren Menge an einzusetzendem Enzym auch sichergestellt werden kann, dass keine unerwünschten Nebenreaktionen im Magen-Darm-Trakt von Tieren auftreten.

Gemäß einer bevorzugten Weiterbildung der Erfindung ist der Futtermittelzusatz so ausgebildet, dass eine Carboxylesterase Sequenz ID-Nr. 9, eine Aminotransferase Sequenz ID-Nr. 19, eine α-Ketosäure als Cosubstrat und ein inerter Träger enthalten sind. Indem der Futtermittelzusatz eine Carboxylesterase, eine Aminotransferase und eine α-Ketosäure als Cosubstrat neben einem inerten Träger aufweist, gelingt es insbesondere, Fumonisine, welche in den Futtermitteln enthalten sind, zuerst zu hydrolysieren, indem von den Fumonisinen Tricarballylsäurereste mit Hilfe einer Carboxylesterase abgespalten werden, und das so gebildete hydrolysierte Fumonisin in weiterer Folge unter Einwirkung der Aminotransferase und der α-Ketosäure als Cosubstrat, im vorliegenden Fall bevorzugt Brenztraubensäure, weiter umzusetzen, indem eine Aminogruppe von dem hydrolysierten Fumonisinmolekül durch eine Ketogruppe ersetzt wird, so dass ein für Tiere völlig ungefährliches 2-Keto-hydrolysiertes Fumonisin entsteht, welches unverändert ausgeschieden werden kann, und als Nebenprodukt Alanin gebildet wird, welches vollständig unschädlich ist und ebenfalls keinerlei negative Eigenschaften auf den Organismus ausübt, so dass ein vollständiger Abbau von Fumonisinen zu unschädlichen Substanzen sichergestellt ist.

Schließlich zielt die vorliegende Erfindung auf die Verwendung eines Gens mit der Sequenzen ID-Nr. 8 zur Expression der Enzymsequenz ID-Nr. 9 ab. Auch zielt die Erfindung auf die Verwendung des Enzyms der Sequenz ID-Nr. 9 sowie gegebenenfalls des Enzyms Sequenz ID-Nr. 19, sowie einem Cosubstrat zur Herstellung eines Futtermittelzusatzes für Tiere ab. Mit derartig hergestellten Futtermittelzusätzen gelingt ein vollständiger und zuverlässiger Abbau von Fumonisinen im Verdauungstrakt von Tieren, wobei für einen derartigen Abbau im Gegensatz zu aus dem Stand der Technik bekannten Genen keinerlei molekularer Sauerstoff erforderlich ist.

In bevorzugter Weise werden gemäß der vorliegenden Erfindung ein Cosubstrat, das eine α-Ketosäure ist, und ein inerter Träger eingesetzt, mit welcher Verwendung Fumonisine sicher und zuverlässig im Verdauungstrakt von Tieren zur Gänze zu unschädlichen Bestandteilen abgebaut werden können.

Die Erfindung wird nachfolgend anhand von Ausführungsbeispielen sowie Figuren näher dargestellt. In diesen zeigt:
Fig. 1 den Fumonisin-katabolischen Gencluster,
Fig. 2 die Michaelis-Menten-Kurve für Fumonisin-Carboxylesterase FumD, und
Fig. 3 eine Abbaukurve von hydrolysiertem Fumonisin B₁.

In Fig. 1 ist ein Fumonisin katabolischer Gencluster als Teilsequenz von 15420 Basenpaaren eines mikrobiellen Stammes mit der Hinterlegungsnummer DSM 16254 dargestellt. In dem fum-Gencluster des bakteriellen Stammes DSM 16254 wird die Transkription der offenen Leserahmen durch einen bidirektionalen Promotor gesteuert bzw. geregelt, welcher zwischen *fumA* und *fumI* angeordnet ist. Der Cluster codiert Proteine, welche in der Regulierung der Genexpression, wie beispielsweise FumB und FumC, in der Substraterkennung und dem Transport, wie beispielsweise FumJ, FumA und FumG und in einem Substratkatabolismus, wie beispielsweise FumD, FumE, FumF, FumH, FumI und FumK involviert sind.

### Beispiele

### Beispiel 1: Die Enzymkinetik von Fumonisin-Carboxylesterase

Das *fumD* Gen (Sequenz ID-Nr. 8), welches eine Fumonisin-Carboxylesterase codiert, wurde kloniert und in *Pichia pastoris* unter Verwendung von Standardverfahren exprimiert. Das his-getaggte Enzym wurde rückgewonnen und aus der überstehenden Lösung der Kultur durch Affinitätschromatographie gereinigt. Die Enzymkonzentration wurde bestimmt und die enzymkinetischen Parameter wurden mit sieben unterschiedlichen Substratkonzentrationen im Bereich zwischen 50 µg bis 25 mg FB₁ pro Liter und einer Enzymkonzentration von 0,33 ng/ml bestimmt. Die Reaktionen wurden in 20 mM Tris-Cl (pH 8,0) Puffer mit 0,1 mg/ml Rinderserumalbumin gepuffert und bei 30 °C inkubiert. Proben wurden nach 0, 30, 60 120 und 240 Minuten Inkubation genommen und durch HPLC-MS/MS analysiert. Fumonisin B₁ (FB₁) und hydrolysiertes Fumonisin B₁ wurden quantifiziert basierend auf einer Kalibrierung mit den gereinigten Referenzsubstanzen und einem vollständig ¹³C markierten, internen FB1 Standard.

Fig. 2 zeigt die Michaelis-Menten-Kurve für die Transformation von Fumonisin B1 (FB1) durch Fumonisin-Carboxylesterase FumD, welche bei einer Enzymkonzentration von 0,33 ng/ml in Tris-Cl-Puffer (pH 8,0) bestimmt wurde, wobei anfängliche Enzymgeschwindigkeiten gegen die Substratkonzentration aufgetragen wurden. Die Michaelis-Menten-Kurve zeigt einen Abfall bei höheren Substratkonzentrationen, da die Enzymgeschwindigkeit basierend auf dem Produkt, d.h. hydrolysierter FB₁-Bildung berechnet wurde. Da hydrolysiertes FB₁ aus FB₁ in einer zweistufigen Reaktion über partiell hydrolysiertes FB₁ mit lediglich einer Tricarballylsäure-Seitenkette, die zurückbehalten wurde, und einer Seitenkette ausgebildet wird, die abgespalten wurde, war die Endproduktbildung bei hohen Substratkonzentrationen verzögert. Die Michaelis-Menten-Konstante K_{M} wurde als 0,90 µmol/l berechnet, was 650 ppb äquivalent ist, und die Umwandlungszahl war 900 pro Sekunde.

Aus Fig. 2 ergibt sich, dass Fumonisine mit der Carboxylesterase in den relevanten Konzentrationsbereichen rasch und vollständig hydrolysiert werden können.

### Beispiel 2: Die katalytische Aktivität von hydrolysierter Fumonisinaminotransferase

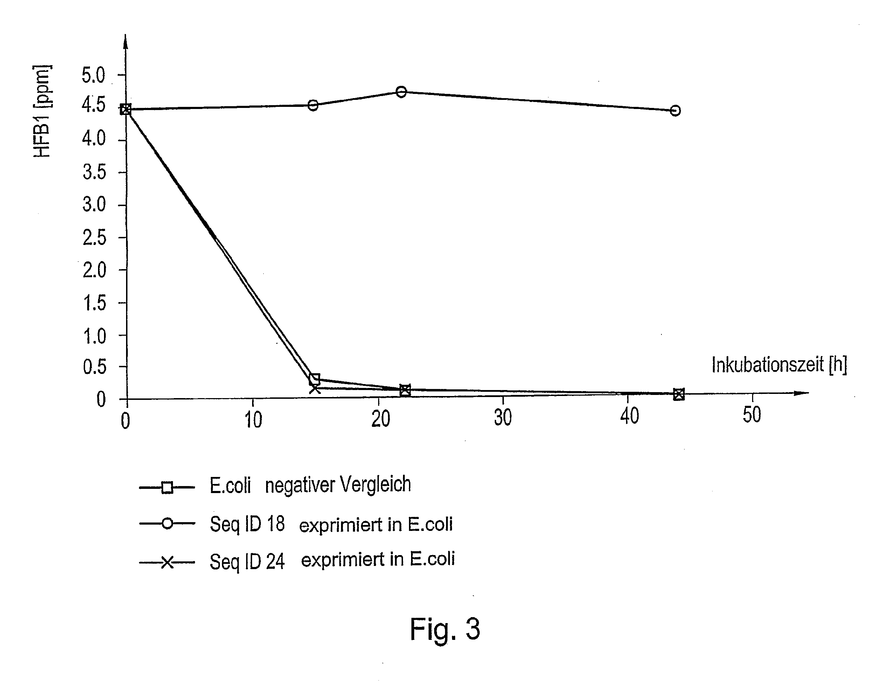

Sequenz ID-Nr. 18 wurde unter Verwendung von Standardverfahren kloniert und in E. coli exprimiert unter Steuerung bzw. Regelung eines Bakteriophagen T7 Promotors. Die Bakterienzellen wurden gesammelt in 50 mM Natriumphosphatpuffer, neuerlich suspendiert und durch Ultraschall lysiert. Hydrolysiertes Fumonisin wurde hinzugefügt und die Proben wurden bei 25 °C inkubiert. Proben wurden in Zeitintervallen genommen und durch HPLC-MS/MS analysiert. Es wurde keine Reduktion der hydrolysierten FB₁ Konzentration beobachtet. Wenn ein Cosubstrat, wie beispielsweise eine α-Ketosäure, wie Brenztraubensäure oder Oxalacetat zu der Reaktion hinzugefügt wurde, konnte ein vollständiger Abbau des hydrolysierten Fumonisins zu 2-Keto-HFB₁ beobachtet werden, wie dies in Fig. 3 dargestellt ist. Diese Substanz ist für Tiere vollständig unschädlich.

### Beispiel 3: Enzymaktivität in Darmmilieu

Zur Überprüfung der enzymatischen Aktivität von FUM-Carboxylesterase im Verdauungstrakt wurden schlachtfrische Schweinedärme verwendet und unter Ausschluss von Sauerstoff ins Labor transportiert und in einer anaeroben Sterilwerkbank untersucht. Etwa 10cm lange Stücke von Duodenum und Jejunum wurden abgebunden und herausgeschnitten. Mit Kanülen wurde Fumonisin B1 in konzentrierter wässriger Lösung auf eine Endkonzentration von etwa 10 ppm eingespritzt und mit Darminhalt vermischt. Anschließend wurden 5 µg Fumonisin-Carboxylesterase in wässriger Lösung, bzw. dasselbe Volumen Wasser in den Negativkontrollen, eingespritzt und eingemischt. Die Darmabschnitte wurden bei 39 °C inkubiert. Mit Hilfe von Kanülen wurden Proben gezogen und durch HPLC-MS/MS analysiert. Dabei zeigte sich, dass Fumonisin B1 im Duodenum und Jejunum zum Zeitpunkt der ersten Probenahme nach zwei Stunden bereits vollständig hydrolysiert war.

### Beispiel 4: Ermittlung des Temperaturbereichs der Aktivität von Fumonisin-Carboxylesterase

Zur Ermittlung des Temperaturbereichs, in dem Fumonisin-Carboxylesterase aktiv ist, wurden 1,6 ng/ml FUM-Carboxylesterase in 20 mM Tris-Cl Puffer, pH 7,0, mit 0,1 mg/ml BSA und 10 ppm Fumonisin B1 bei unterschiedlichen Temperaturen inkubiert. Dabei zeigte sich, dass das Temperaturoptimum für das Enzym bei 30 °C lag. Auch bei 40 °C und sogar 50 °C wurde enzymatische Aktivität noch eindeutig festgestellt. Somit ist diese FUM-Carboxylesterase zur Anwendung unter den Temperaturbedingungen wie im Verdauungstrakt, oder im Zuge von Prozessschritten der Lebens- oder Futtermittelherstellung, die bei erhöhter Temperatur stattfinden, geeignet.

### Beispiel 5: Bestimmung des pH-Bereichs der Aktivität von Fumonisin-Carboxylesterase

Zur Bestimmung des pH-Bereichs, in dem Fumonisin-Carboxylesterase aktiv ist, wurde Teorell-Stenhagen-Puffer verwendet. Dieser Puffer lässt sich durch die Kombination von Citrat, Phosphat und Borat über einen Bereich von 10 pH Einheiten mit gleicher Pufferkapazität einstellen. FUM-Carboxylesterase wurde in einer Konzentration von 3,3 ng/ml mit 10 ppm Fumonisin B1 bei verschiedenen pH-Werten in diesem Puffer bei 25 °C inkubiert. Die höchste Aktivität zeigte sich bei pH 8,0, aber es konnte im gesamten Bereich von pH 5 bis pH 10 Aktivität festgestellt werden. Durch die Aktivität in diesem breiten pH-Bereich wird die technologische Anwendung des Enzyms als Futtermittelzusatz bzw. in Zuge der Verarbeitung von Lebens- und Futtermitteln ermöglicht.

### Beispiel 6: Fütterungsversuch mit Ferkeln

Der Versuch wurde in einem Versuchsstall mit 12 Buchten für jeweils 10 Tiere durchgeführt. Der Stall war ausgestattet mit Spaltenboden, Schalentränkern und einem computergesteuerten Fütterungssystem. Die Automaten waren entlang der Buchtenwände angeordnet. Das Stallklima wurde täglich automatisch aufgezeichnet und die Temperatur nach den Standardempfehlungen zur Ferkelaufzucht eingestellt.

120 gemischt-geschlechtliche Absetzferkel (Alter: ca. 4 Wochen, durchschnittliches Einstellgewicht 8,21 kg) wurden für diesen Versuch eingesetzt. Jedes Ferkel wurde mit einer Ohrmarke versehen und einzeln gewogen. Die 120 Ferkel wurden auf 12 Buchten zufällig verteilt. Alle Ferkel entstammten dem österreichischen Zuchtprogramm ÖHYB (= (Edelschwein x Landrasse) x Pietrain).

Direkt nach dem Absetzen wurden die Ferkel für 2 Tage mit einem Starterfutter gefüttert, nach dieser Eingewöhnungsphase erfolgte die Umstellung auf das Versuchsfutter. Die Fütterung erfolgte 2-phasig: Absetzphase Tag 1 - 14, Aufzuchtphase Tag 15 - 42. Das Versuchsfutter wurde buchtenindividuell über die Spotmix-Fütterungsanlage gemischt und je nach Anzahl der Ferkel, Gewichtsentwicklung und Futterverzehr zweimal täglich trocken zugeteilt. Wasser stand zur Aufnahme ad libitum zur Verfügung. Die 12 Buchten wurden in vier verschiedene Applikationsgruppen zu je drei Wiederholungen geteilt und erhielten die folgenden Einmischungen ins oben beschriebene Futter:

| Gruppe | |
|---|---|
| Negativ-Kontrolle | Keine Toxine, kein Enzymzusatz |
| Positiv-Kontrolle | 4 - 5,5 ppm Fumonisin B1 |
| Versuchsgruppe 1 | 4 - 5,5 ppm Fumonisin B1 + Enzymmix 1 (Carboxylesterase, Aminotransferase, Pyruvat) 0,5 kg/t Futter |
| Versuchsgruppe 2 | 4 - 5,5 ppm Fumonisin B1 + Enzymmix 1 (Carboxylesterase, Aminotransferase, Pyruvat, inerter Carrier) 1 kg/t Futter |

In der Positiv-Kontrolle wurden bei fast der Hälfte der Tiere respiratorische Probleme beobachtet, wobei es auch zu einem Ausfall kam. Alle anderen Gruppen erschienen gesund.

### Leistungsdaten

| Gruppe | Anzahl der Tiere | Anfangsgewicht (Durchschnitt, kg) | Endgewicht (Durchschnitt, kg) | Ausfälle |
|---|---|---|---|---|
| Negativ-Kontrolle | 30 | 8,34 | 26,82 | |
| Positiv-Kontrolle | 30 | 8,17 | 24,77 | 1 |
| Versuchsgruppe 1 | 30 | 8,08 | 26,69 | |
| Versuchsgruppe 2 | 30 | 8,25 | 27,03 | |

Organization Applicant
   Street : Industriestraße 21
   City : Herzogenburg
   State :
   Country : Austria
   PostalCode : 3130
   PhoneNumber :
   FaxNumber :
   EmailAddress :
<110> OrganizationName : Biomin Holding GmbH
   Application Project
<120> Title : verfahren zur Herstellung eines Futtermittelzusatzes zum sauerstoffunabhängigen, enzymatischen Abbau von Mykotoxinen sowie Futtermittelzusatz und Verwendung desselben
<130> AppFileReference : P04568
<140> CurrentAppNumber :
   <141> CurrentFilingDate : __-_-_
   Earlier Applications
<150> PriorAppNumber : AT GM502/2008
   <151> PriorFilingDate : 2008-09-18
Sequence
<213> OrganismName : Sphingopyxis sp.
<400> PreSequenceString : 
tgtcggcgat crgtaaactt ctaccgtggt cctcgttcgc ccacakcata catcacagac 60
rtcgggattt ccaactgaac gggtcccggc ctgccggccc acatttcccg gaacgccata 120
tgggtgattt cgacaatccg gttccaggcg aagatgggtg cgccccattt aaccgcgggt 180
cgaaagaggt cgatctggtc ttgtccctga aaggtttttg gcgtgcaggg ataaacgaca 240
ccaagttgat gctgggacgt tattgcgacg aagggaaccc cttcgtggcg tgccgtcacg 300
actccaggca gaaggtttgc cgtaccggga cccggattcg tgacaatcgc ggcgacctgt 360
ccggtggtct tgtaaatgcc ctcggccata taggctgcgg cggcctcgtg ccgcaccggg 420
acgaacaata tcccattgtc ttcgagcgca gccaggagcg gatccacctc cggcgacatg 480
aggccgaaga cataccggac gccttcgacg gccaaacatc gtgccaataa ttctccgccc 540
gtgaggcgca tgacgatctc cagtacgaaa ggtgagtgcc caggttccgg cacattcgct 600
gtggttagtt gatgcgctga tcggccaacc gactgagtgg agttggatgg ccgcacctta 660
ccctgtcgcg cataactctc agatccggaa acggaccccg acattaaaat agcggccgac 720
cggatcatag gcagagctgg tcggctggaa aaaactgctg gggtcgttcg tcgctattgg 780
cggatctcgg tcgaacaaat tattgaccga tagaaacagc tgctgcttct ggccaaaagc 840
cgcgatgtcg aaggtcaatg tcgcgtcggt gtaccaaacc gccggagcgt ggttcaaatt 900
cgtatcgacg ccctccacat tgtcggcatt gaacaccgat gctgcgatga agcgctgctg 960
cacgagaagc gcccaatcgt cggtcgaata tcgcgcctgg aagttggccg accattttgg 1020
cgtgtccggt tgtccgagcg aacggatggg cgccgagccg gtcgcgatgc gataggcaga 1080
ggtatggtgc gttgccagcg cacgaagact gaacgtgccg ccgccgacgg ggcgtgagta 1140
ataggcctcg aagtcaattc ccgccgcttt ctggacagcc aggttgagat tgggacccgt 1200
cactgtgatg gtgccgtccg gattctccgt tatgaggtcg cagaagaagg tgtttcctgc 1260
atcgcacgcg tcgatttcct gctggggaag gaggaaatcg atcgcgccct tcaccttcac 1320
cacatagcga tcgaccgaaa actgaaaccc cggcacgaag gcggggcgta gcaccgcgcc 1380
gaatgtaagg acgtccgcct tttcagggcg caaatccgcg ttgccggcgg taaagaaccg 1440
cgtctgcaca gcctgtccgc cataaattga attgagcgtc gcctgacggc cggggtcgaa 1500
tagctcgaca aggcttggcc cgcggatatc tcgcgaacgg gtcgcgcgga acctgaggcc 1560
gtcgatcggc tcatattctc cgcccagctt ccaggttgtt actccaccgg actggctgta 1620
atcggcatat cggacggcgc cgtttaagtt cagcgaacgt cccagcgcgc tgtccttcag 1680
aatcgggacg ccgatttcga caaaaccttc cttgatgtca tagcttcccg agaaggaaag 1740
tgggttgtag agattgaagc ctccaggccg acctgcctgc gccgccggag ccccccctgat 1800
tcccgtgatc gaggtcgtcg cctgcgatat cgcgtcggtt tcctgccggg ccttctcctt 1860
gcgatattcg ataccagcgg cgaccgagac cgggcccgcg ccgaacgaca ggctatcgcc 1920
gaggtcgccg gaaatcgtga gtcccgccac atattgctca agcctcagct gagcgacgcc 1980
atcagcggtg acatagtcga tggccgacgc gctcggcgag cctgtgccga agagattgag 2040
cggcacgcaa tcttggtcga ggccggccag tgttgaacgg cagacgatat tgcccgcggg 2100
atcgcggacc gcatcgacgg cggcgtagag attgcggttg atggtgagat tgttttcacg 2160
aagctcgagg tccgtaaggc caaaggaggc cgagccatcg agtttccagc cattgccaat 2220
gtctgcccgg aagccggcag cgccgcggta gacctttgcg aaattctcga tttcgaccaa 2280
 
gggaaagtcg cttgagaagc gaccgacaac gatcgaagcc tgggcatttc tgtccatgag 2340
cgtcgcgagt ggagccggaa ggaaggcgtt atcacggaag atccggaaat tattcgagcc 2400
accgacatgc gatattacga atgcacccag gttggtgtgg gaataagcat aggtgccctc 2460
cgcatacacc tgcacagtgt cggacacatc atatgcggcg cgtaggaacg cgttgtagcg 2520
aagctgatcc ggggcgaagc cgatattcac gcgcggtcca tcgccgccgc tctggaacga 2580
cgagctcgta aaattcccgt agtcgaaggt ccctaggact cctccgggca aaaacgcgat 2640
gcctttcaga gggccggacg tgacaagtcc gccgtaggat ccgcgagaac tgcgaatatc 2700
gggcacgacc gtgacgcctg tcgtagcgcc gggcacggga tattggccgg cggcgatgtc 2760
gaaccagcgg cgacccgttg cttcatcggc ccggattccg tcctgtcgaa aatattcgaa 2820
gctgccgagc aagtgcaacc ggtcgtcggc aaacgaagtg ccgaaggcga tcgaaccgcc 2880
gtaggacggg aggtcgccgc gggttgaaac acccgactgg agctcggccc tgatgccttc 2940
cagatcttcg tcgagcacga agttgatgac gcccgaaacg gcatcggaac cgtaggcggc 3000
cgaggcgccg cccgtcacga catcgacgcg cttgaccaac gcctgcggca gcacgttgat 3060
atcgaccgag cctgtgaaat tggtcgcgac gaaacggttg ccgttcagca ggacgaggtt 3120
ccggtttgac ccgaggccgc gcatgttgag caggttctga ccgctgttcc ccgttccggg 3180
tgtcgtgcca gggttggagg tcttcaagct gtcgttgaac acgggcagct ggttgagtgc 3240
gtcggcaagg ttggtcggag atgcctcctt caactgctcg ctggatacgg ctgtaaccgg 3300
cgtcggcgaa ttgaagccgt tctggaggcg gctgccggtc acgacgattt cgctcgttcc 3360
ccggtccgtg tccgcttcgt ccggctgacc tatcgatgcg ggatcgctat cctgagcact 3420
ggcagagaca ggaaatgcga gggtgccgag cgctactgcg ccgagcaaac tatttgcctt 3480
gccgggcttt tcgattctga acttccgata catctgcagt ccctcccgaa ttgataggga 3540
ctccgtttga gtccccttgt ttcttgacgc cgccgtcgct caccacggtc cggtcggagg 3600
ctaagcgtcg ggcctaagga cccgcaattt gaacatcaaa tgcaatgatc ggaggcttca 3660
ttgcacttcg cgcatagacc ggcgcggtag ctgaaagtgc caataatcag ggattttgct 3720
gaacagttgc ggcatgacgt ccggcatcgg ccacgcggtt ggcggcatcg acgtggcttt 3780
cgcgtcgccg cccctcaagc accggcgagt tgcattaaaa tgggatgagg ctggagagac 3840
gcaaaatctc tgaggaccgc gctgaacgcg cgatccgtcg cctcgagggt ctccgttaca 3900
tcgtcaactg tatgggccgc agagagaaac atattgtgat agggatgaac atagacgccg 3960
cccttcaggc acgccgcggc ccacgcatag ccgatccgaa aatcgggatc gtccgcaaag 4020
aatatttgcg gcatctgcgc cgggcccgtc tgcttcaact caagaccatg gcgctgagac 4080
tgtgcctcca ggcctgcccg cagggcggcg ccgctggcga tcagcgtttc gagataaggc 4140
gtctctcgaa tgatcctgag ggtttcgatc gcggccgcca tcggtaccgc agagaaccag 4200
aaggagccgg tcacaaatat atcccgcgcc gcatcgcgcg ccttgttcga gcccagcagg 4260
gcggagatcg gatagccatt cgcaaagcat tttccccagc aactgagatc gggttcgata 4320
cccaaatgcg tccagctgca atcgcgcgcc acccggaaac ctgcgcgcac atcgtcaacg 4380
accagaagcg caccggtctc gtcacaacat tttcgagcgg tgcgcgcgaa ctcaagctgg 4440
gcgagggcct ggtcctcaaa tacttcgtgt cggaaaggtg tggcaaagac agccgcaata 4500
tcgccatcgt gcgccttgaa cgcgtccgat aagctttggg cgtcgttata ggtataatat 4560
gcgacatgca cgcgatcgga agcgagaatc ccggcagtat gcggagtgtt ccacggggaa 4620
gcgccatgat aggcgccttt ggcgcataat atggttttgc gccccgtatg ggcacgcgcg 4680
agaaccatcg ccgttgaggt ggcatcgctg ccatttttgc agaacatcgc ccaatccgca 4740
tgacggacca tgcccacaaa ggcttcggcg aggttgacca tgatctccga aggaccggtc 4800
atggtgtcgc cgagaagtcg ctgcgcatca gccgcggctt cgatttcgga ttgccggtaa 4860
ccgagcaaat ttggcccata cgcgcacata tagtcgatat agggctgctc gtcggcgtcc 4920
caaattcgtg cccccagcgc gcgcctgaag aactggggga attctggcgg cagcaaccgt 4980
gtcgactcgt ggccgtacat cccgcccgga atgacccgtt cggcgcgttc tctgagatct 5040
ttctgccttg ttccgttcgc cataatgcac ctctcgcgat aaataatggg taaaaatcca 5100
cgaaattcaa cgattcgtga tctgaaagag atatatcttg taatatactg tataattata 5160
cacaatgcgc aatcggacga cgggatagcg ggcagggagg gacggggaaa tctatgcgga 5220
acgtcagcga caaggcgccg ccccacgaga cgctcaccgt agtcgtcgcg gcaatgatcg 5280
ttggcacggc cgccttgatg gtgcttggaa tacagcccat ccttctcggc gcccttgtag 5340
aggaggggcg tattcccgcc gaggggttgg gatcggcggc aacggtggaa atactggcga 5400
tcgcggcggg aacatgcatc ggacccgttc ttatgaagac gggatatctg cgggcgaaat 5460
gcgcggcact ctgcttaatg ctcgccgcaa tcaacttcgg attgacgttg ccgggtttcg 5520
atttgcccat cgtggcttgc cgagcggcag cgggagccct ggaaggtctt tcgctcagcg 5580
cggcgatcct gatcatgact cataatcggc ggccggaccg gctgagcgga atatttctgg 5640
gcgcgcagac gataccgcag gtaatatctg cttatttgct cccgacggag attattccgc 5700
gctgggggag cgcaggcggc ttcacgatcc tgggcattct cgcggcgatc gccgcgatcg 5760
cggctctgtg cctcgtcgat cgcgttgagc tcgatccgac gaccgttaac gacgacttgc 5820
agtggtcacc cgcggcgatc gtcatttcga tggcggcatt cgttcaattc tcgggggtcg 5880
gtgccgcatg gagctatctg gagcgactgg ctgcgcagca cggattttcg ggagaaacga 5940
tcggtatcgc catttccggg agtttgcttt gccaggtagg cggggcttgg ctggccgctt 6000
ggatcggtgg gcgggtcgga tatcgcttcg ccttaatcgc tgggagcctg cttcaggcgg 6060
gcaacgtgat cgcattggcg gtggccgatc agccaagctg gtttatttcc gcttcctgtg 6120
ctttcggcct gttctggttg gcgatgcagc ccttccaaat ccgcttcgcg atcgcgatag 6180
ataacagccg gcagcttgct gtactgctga cgccgatcgc cctcgtcggg ttgagcgcgg 6240
ggcccttgtt gctctctcgc tttgccgggg cgaccgactt gcgctggatc tttgtgggga 6300
gttcgacctt gttgctggcc agcgcgcttc tgtatctttg cgcttctctg tttcaaccgc 6360
 
gcggaaaggt gatcgctgaa acggtggacg tatgaaaaag acggatcggg gttcgcgatg 6420
acatcgcagg tcaagcttcg tagcgcggca aagcggccgc gcagtcctaa aagcgagcga 6480
ggtcttgctc gttacgagtc cttgcttgat gcgaccgaca ggctgttggt cgatctagac 6540
cccgatcagg tcggtctcta tcagattgca gaggaagcgg gtgcctcacc gtcgtccgtc 6600
tatcatttct ttccgaccaa ggaagtggct catctcgctc tgatgcgccg ctatctggag 6660
gggctccgga atctcgacgc gatggaagtc gacatcggcc agctcgaaag ctggcaggac 6720
ctgatgaagt tggatcagat cagggcgcga gactattata atagccaccc gcccgccctc 6780
aagcttctgt tcggcggata tggcggggtc gaggccagaa agcttgacga gcgatactcc 6840
gaggaaatcg tgagctccat gtatggcaga tacaacggca ttttccatat gccgcaaatg 6900
gagaatgagg ctctcatgtt cacgatctgc ttcgcaattc tcgacgcggt atgggccgtc 6960
tcctttcgcc ggttcggtga aattacgtcg gattttcttc gggaggggca agcggcttgc 7020
attgcctatt gccgacacta tctgcccgag cgaacgccat cagcgtgaat ccgttcaacg 7080
atatgcagga atgtccgttg cgttgagttc ggttctgagt tcggtcggtt aggaggcccc 7140
gcgataaacc aacgctcttc tgtcgaaggg atgtcgcctg gttcgaccag gccctgcgaa 7200
gtcagccgca atcaacgagg cagatgtcaa cgtggccagc aagttcaact gtgagttact 7260
cgatctgcga tcatttgttg cggtgtatga aacgcgaagt tttagccacg ccgcgcggct 7320
tctgaatcaa tcgcagcccg cgctcagccg gagaatccag cgcctcgaga gtctcgtggg 7380
cggtccgttg ttcgagcgga ccagtcggtc gcttgccgaa acggcgctcg gcaaagagtt 7440
gctcccggtc gcccaccgag cgttggaact tgtcgatacg tcgctgtttg cgtcgcccaa 7500
tgtccgggag ttccgctgga cagacatcac gattgcctgt gtacagaccg ccgccttcca 7560
tgttctcccg cgagctgcgc gcttgtacat ggatcaaaat ccgagggtcc gactccgcat 7620
ccttgacgtg ccggcggtcg aggctgcgga cctggttgcg agcggcgagg cggagttcgg 7680
catcagcatt gagagcctgt tgccatcaag cctgcggttc gatgcgctcc acgaggaccc 7740
gttcggcctg gcatgccacc gaagccatcc gctggcgtcg ctcgagatcc ttgaatggac 7800
gcaattgaaa ggtgaaagcc tgatcgccgt tcaccgtgcg agccggaacc gcacgttgct 7860
cgatgccgaa ctcgcgcgca acaatatcgc gctggaatgg cggtatgagg tcgcgcatct 7920
gacgacggcg ctgggattga tcgatgcgca attgggtgtc gctgttatgc cccgcatggt 7980
tatgccccgc tcgggtcggt cggaggtcgt ctggcgcccc gtcgtcgcgc cggtcgtcca 8040
acgcacgatc ggcatcgttc agcgccgcac cggctcgatg caccctgccg cacagcaatt 8100
gcttgcgcgg ctccgcgcgg cctggtcgtc cgccaatctg ggcgacatcg cgtctcgcga 8160
agatggggca tcgtgacacg cgttctatgc gcctgcagca tcgatgctca cgatcattgc 8220
atttgctgag agacgaacgc gaagataccg ctgggtcaca ggatatcagt ccatcgaggc 8280
gggagagaaa tgtgtgaaag agcaccaatg ccgtggcggc cgggcgtccc ccgctgcgcc 8340
cgccacgtgg cttgcgcgga tcagcgtttc ccgggggccc tccgccatcg cctggacctt 8400
catgcttggc gcaactgcca ttcccgtggc tgcgcaaact gacgatccga agctcgttcg 8460
tcatacccag tcgggcgccg tcgagggcgt cgagggcgac gtcgagactt ttttgggaat 8520
acccttcgcg gctccgccgg tcggcgacct gcgatggcgg ccgccggctc cgccgagggc 8580
gtgggcgggc accagggacg gccgccgctt tgcgcccgat tgcatcggga acgagcggct 8640
tagagagggg agccgggctg ccgggacgag cgaagactgc ctctatctga atatctggtc 8700
tcccaaacag gtcggtaagg gggggctccc cgtcatgatc tgggtttacg gcggtgggtt 8760
tagcggcggt tctggcgcgg tgccatatta tgacggctct gcgctcgcgc agaagggcgt 8820
ggtggtcgtc acgttcaact atcgcgccgg gattctgggc tttcttgccc atccggcgct 8880
ttcaaaggaa agtccgaatg gcgtgtcggg caactatggt cttctcgaca tgctcgcggc 8940
gttcaaatgg gttcagaaca acataaggga gttcggcgga gacccgaacc gtgtcacggt 9000
ctttggcgag tccgccggcg cgagcgcgct cggactgctc ctgacctcgc cgctcagtga 9060
gagcgccttc aatcaggcga tactgcaaag tccgggtctg gccaggccgc tcgccacgct 9120
ttctgaaagc gaagcgaatg ggctggagct gggagccgat atttctgctc tacggcgtgc 9180
cgatgcgggc gaattgacga agatcgcgca atcgcgaata cccatgtcgc gccagttcac 9240
caagccgcgg ccgatgggtc cgattctgga cggctatgtt ttgcgcaccc ttgacgtcga 9300
tgccttcgcc aagggggcct tccgcaagat acccgttctg gtcggcggaa acgccgacga 9360
agggcgcgct tttacggatc gcctgccggt caaaacggtc cttgaatatc gagcctatct 9420
cacagaacaa tttggtgacg aggcggacgc atgggagcgt tgttatcccg cgaactccga 9480
cgccgacgtc cccgccgccg ttgcccgtct tttggggat agtcagttca acaacgggat 9540
cgagctgctc tcggcagcct tcgcgaaatg gcgaacgccg ctttggagat atcgctttac 9600
gggcattcca ggagccggcc gtcgccccgc cacgcatgga gacgaaattc cctatgtctt 9660
cgcaaatctg ggccgtcgt ccgtatctat gtttgggtcg ctcgaaggcg gcgccggggc 9720
gtcggacatc aaacttgcga ccgaaatgtc cgcggcctgg gtgagcttcg cggtgcacgg 9780
ggtccccgat cagggcacga aatcgcactg gccgcgcttc gagcggcgag ggagatcat 9840
gacttttggt tcgcaggttg gctctgggga aggtcttgga gtttcgccga gcaaagcctg 9900
ccaaccctca aaatagcgcc cggcctgtgc gtgcttcagc acgccgtccc gctttgcggg 9960
cgacgggctg tgccctctgc ctagaaggaa gtaagttgcg ctacgacgtc gcgataattg 10020
gaggtggcaa cgctgcattg acggcagccg tgacggcgcg tgaagcgggg gcctcggttc 10080
ttgtgatcga gcatgcgccg cgcgccatgc gcggcggcaa cagtcgtcac acacgcaata 10140
tgcgtacgat gcacgaacgt cccctgtcgc cgttgaccgg tgaatattcg gcggacgaat 10200
attggaatga tcttgtccgc gtcacggggg ggcgcaccga cgaagaactc gcgcggctcg 10260
ttatccgcaa caccaccgac gctattccct tcatgacgcg ctgcggtgtg cgtttccagc 10320
cctcgctgtc gggcacgctg agtttatcgc gaaccaacgc attcttcctt ggcggcggga 10380
aggcgcttgt aaacgcatat tacgccacgg ccgaacggct aggcgtcgat attctctatg 10440
 
attctgaggt gaccgagatc aaccttcagc aaggcgtcgt gcagcgtctg caattgcgca 10500
gccggggatt ccctgtcgaa gtggaagcca aggctgccat cgcctcgtcc ggaggattcc 10560
aggcaaatct tgactggctc tcaagcgcat gggggcctgc tgcggcgaac ttcatcgtac 10620
ggggcacgcc atatgcgact ggcacggtgc tcaagaacct gttggagcaa ggcgtcgcct 10680
cggtgggaga tccaacccaa tgccatgctg tcgcgatcga tgggcgagcg cccaaatacg 10740
acggcggcat cgtcacacga ctggactgcg ttcccttctc gatcgtcgtc aacaaggacg 10800
ccttgcgctt ctacgatgaa ggcgaagatg tgtggccgaa gcgttacgcc atatggggtc 10860
gcttggtggc acagcagcct gatcagatcg ctttcagcat aatcgatcgg caggccgaag 10920
acctcttcat gccgtcagtg ttcccccccg tgcaagcgga cacgatcgcg ggtctggccg 10980
agaaactcgg tctgaatccc gtaaccctgg aacgcacggt ggccgaattc aacgccgcat 11040
gcgtgcccgg cgaattcggc ggccaagatc tcgacgacct ccacaccgag ggaatcgaac 11100
caaagaaatc caactggccc cgaccgatta ttgtgccccc gttcagcgcc tatcctctcc 11160
ggcccgggat caccttcacc tatctcggcg tcaaggtaga cagccgtgcg cgggtcatca 11220
tggagacagg tgagccgaca aaaaacctgt ttgcttcggg ggaaataatg gcgggcagca 11280
ttctcggcca aggttatctc gctggatttg gaatggcgat tggtaccgta ttcggccgca 11340
tcgcgggttg ggaggccgca cgtcatgcag gattttgatc tcgtaaaaat gctgtctgac 11400
ttgccgtcgg cgccggagct ggaagccagg cgcgttatgg aggtgtgcaa cgcgtgccgc 11460
tattgcgaag ggttctgcgc ggtatttcct gcaatgacct tgcagcgtca tttcgccagc 11520
ggcgatctca gccacctcgc caatctctgc cactcgtgcc aaggttgcta ttacgcctgc 11580
caatacgccc ctccgcatga gttcggaata aacgttccaa aggcgctgtc ggagttgcgg 11640
ctcgagagct acgagcagca tgcttggccc cggccggtcg ccgctctcta tcgcaagaat 11700
gcgctcatca tttccatctt gtcggcggca tgcataaccg gcgtccttct gcttgccgcc 11760
atcttcaacg ggatgcact tttcgcgaaa cacgcatcgg tgcccggcgg cgggttttac 11820
aacgttattc cttatcaggc gatgattgcc gtcgcggcga ccacatttct ttattccgcg 11880
ctggcgctgg cgatcagtct cgttcgcttt tcgcggacga tcggtctggg aattaaggtt 11940
ctttatcagc acgtgccggt tcttcgggcg ctacgcgatg cggcgactct gcgatatctc 12000
ggcggcagcg acggcgaggg gtgtaacgac gcggacgaga catttcgac gacccggcga 12060
aaatttcatc acgcccttgc ctatggcttc ggactttgtt tcgcggccac agccacgggc 12120
acgatctacg atcatatgtt cggctggccg gcgccctatg cgcttttcag cttgccggtc 12180
gtcctaggga ccgttggggg gatcggaatg gtcgtgggcg cgatcggcct actctggctc 12240
aagctggccg gcgaagacgc tcctcgatca ccggcactgc ttgggccgga tgttgccctg 12300
ttggtgcttc tgcttgccat agcggcaacg ggcctcctcc ttttagcggt ccgcagcacc 12360
gaagtcatgg gcgtcgcgct cgccgtccat ctcggcgtcg tcttggcctt ctttttggtg 12420
atgccataca gcaaatttgt ccacggtatc ttcaggctca cggctctcgt gcgccatcat 12480
gctgaccgcg aggcaagtaa tggcttcgcc tccagccctc ccacgaaaaa gggttaaaca 12540
atggaacata tgaagtccgt tcgcgatcgc agtagcgtca tgcagatcgt gagagtggcg 12600
agtggcaact gtctcgagca atatgatttc ttcgtttacg gcttctatgc ggcatatatt 12660
gcgagaagct tttttccgac cggcgataac gcgacatcgc tcatgctttc attggccact 12720
tttggcgctg gtttcctcat gaggcccttg gggcgatttt ttctcgggtc ctacatcgat 12780
cgcgtcgggc gtcggaaagg cctgatcgtg acactcgcga tcatggccgt cggaaccctc 12840
accattgcga tgactccaag ctatgaggca attggattac tcgcaccggt tatcgtgctc 12900
gtcgggcgac ttttgcaggg tttttccgct ggagcagagt cgggtggcgt ctcagtgtac 12960
ttggcggaaa ttgcgtcgcc caaatcgaga ggcttcttca cctcgtggca gtctgccagc 13020
cagcaggtgg ccgtcatgat cgccgccgcg atcggtcttg cgctgcaatc aacgctttca 13080
ccggagcaaa tgaacgactg ggatggcgg gtgcccttgt tgatcggatg cttgattatc 13140
cccgtgatac tctggctgcg ccggtctctc ccggaaacga aagcctatct ccacatggag 13200
cacaaggcgc attcgatcgg cgaatccctc cgcgaattgc aacagagctg gggctgatc 13260
ttgacgggca tggcgatgtc gatcctcacg acgaccacct tttacatgat taccgcctat 13320
acgccgacat ttggcgagaa agcactcgga ctgagcccgc aagatgtcct gctggttacc 13380
atcatggtcg gcgtgtcgaa cttcctgtgg cttccgatcg gggtgctct ctcggatcgt 13440
atcggtagaa ccccgatcct actggtcgtg ccggtcaccg ttctcgccat cgcctttccc 13500
ctgatgagct ggctcgtcgc ggcaccgaca ttcggagcgc ttgcagctgt tctgctgact 13560
ttctccgcat gctttggact ctataatggg gcgctcatcg cgagactcac cgagattatg 13620
cctcccgcca ttagaaccct tggcttctcg ctggcgttca gtctcgcgac ctcgctgttc 13680
ggcggcttca ccccattggt aagtacggcg ctaatccacg cgacgggcag caattccgcg 13740
cctgcaatct ggctctgttt tgcggctttc atcagcttcg tcggtgtggc cgcatcgacc 13800
cggctgagcc ggccaatcgc cgaaggcgcc agataggaca atcagagaat gcccgtgcgg 13860
caatgaagcg agattcgggc ggtaggtgcg ctggcggcac ttcgcgaaga gccgttgcgg 13920
acggctgaaa cgatgatggt atgaatgggc taagacatga gagcagtagt ttaccgaaat 13980
ggcgaacttg tcctgggggc ctatgctgat ccgatacccg ccgccgggca ggtgctcgtc 14040
aagaccagag catgcggcat ctgcggatct gaccttcatt tttgcgatca tgcgcaggcg 14100
tttacgaacc ttgcatcgcg ggcgggtatc gcctctatgg aagttgattt gtgtcgagac 14160
atcgttctgg ggcatgaatt ctgtggcgag attatggagt tcgggccctc tgcggatcgt 14220
cgcttcaaac ccggacagct tgtgtgctcg ctgccgctgg cgatcggtcc gaccggagcg 14280
cggacgattg gctactcgga tgagtatccc ggcgggctcg gcgaatatat ggtcctcacg 14340
gaagcgctct tgctgcctgt tccgaacggc cttccggcga cctgcgcggc gttgacggag 14400
ccgatggcgg tgggatggca tgccgtcgag atcgcgcagg ttcaaccaca tcacatccct 14460
gtggtgatcg ggtgcggacc ggtcgggttg gcagtcgtcg ctgccctgaa acataagcaa 14520
 
gttgctccga ttattgcgtc ggatccatcg cccgatcggc gtgctcttgc tctgcggatg 14580
ggcgccgacg ccgttgtcga tccgcgcgaa gaatcaccct ttcgccaggc cgagaagatc 14640
gcacgcccgg tcggacaagg tggggccctg tccagctcat tgctgtcaaa gtctcaaatg 14700
atattcgaat gcgtaggggt gccgggcatg cttcggcatg cgatggacgg cgcgtccgac 14760
gggtccgaga tcatggtcgt tggcgcatgc atgcagccgg acgcgatcga gcccatgatc 14820
gggatgttta aagcgctcac gatcaaattc tcgcgaactt acacgggtga ggaattcgcc 14880
gcggtgcttc acatgatagg tgagggcgca ctcgacgtat ctccgctcgt taccgatgtg 14940
attggcctgt ccgatgtccc gtccgcgttt gaggctctac ggagtccagg cgcccaagca 15000
aaagtgattg tggacccttg gcgctgagcc tgaggatgcc aagggtgcga cgttgggcat 15060
cgtcaaagaa ggcgacgttg acccggtatg tgaacatccc catattcttc cgcagctgaa 15120
gcagttggta aacatgccaa aatatgaact gtagtattgc gtcggggttc tcattgtggg 15180
gtttgccatt gtcatcgctc gcacccggcg acaaagatta gatgtacttc cgataatccg 15240
tgctctcgac ctggccttcc ttcatatatt tcaggacctc tccgaccatg cgtgcggcgc 15300
ggatcgggat cggcaggcgt tggttcatct gggtcgagtt ccagttgatc ttcgtaagag 15360
agaacacctc ctcggctaac tgcgccgcgg tactatcgca ggatcgtctc gagcgtycgc 15420
<212> Type : DNA
<211> Length : 15420
   SequenceName : sequence 1
   SequenceDescription :
   Feature
Sequence: sequence 1:
<221> FeatureKey : Seq ID 1 (fum) <222> LocationFrom : 1
<222> LocationTo : 15420
   Other Information :
   CDSJoin : No
Sequence
<213> OrganismName : Sphingopyxis sp.
<400> PreSequenceString : 
atgcggaacg tcagcgacaa ggcgccgccc cacgagacgc tcaccgtagt cgtcgcggca 60
atgatcgttg gcacggccgc cttgatggtg cttggaatac agcccatcct tctcggcgcc 120
cttgtagagg aggggcgtat tcccgccgag gggttgggat cggcggcaac ggtggaaata 180
ctggcgatcg cggcgggaac atgcatcgga cccgttctta tgaagacggg atatctgcgg 240
gcgaaatgcg cggcactctg cttaatgctc gccgcaatca acttcggatt gacgttgccg 300
ggtttcgatt tgcccatcgt ggcttgccga gcggcagcgg gagccctgga aggtctttcg 360
ctcagcgcgg cgatcctgat catgactcat aatcggcggc cggaccggct gagcggaata 420
tttctgggcg cgcagacgat accgcaggta atatctgctt atttgctccc gacggagatt 480
attccgcgct gggggagcgc aggcggcttc acgatcctgg gcattctcgc ggcgatcgcc 540
gcgatcgcgg ctctgtgcct cgtcgatcgc gttgagctcg atccgacgac cgttaacgac 600
gacttgcagt ggtcacccgc ggcgatcgtc atttcgatgg cggcattcgt tcaattctcg 660
ggggtcggtg ccgcatggag ctatctggag cgactggctg cgcagcacgg attttcggga 720
gaaacgatcg gtatcgccat ttccgggagt ttgctttgcc aggtaggcgg ggcttggctg 780
gccgcttgga tcggtgggcg ggtcggatat cgcttcgcct taatcgctgg gagcctgctt 840
caggcgggca acgtgatcgc attggcggtg gccgatcagc caagctggtt tatttccgct 900
tcctgtgctt tcggcctgtt ctggttggcg atgcagccct tccaaatccg cttcgcgatc 960
gcgatagata acagccggca gcttgctgta ctgctgacgc cgatcgccct cgtcgggttg 1020
agcgcggggc ccttgttgct ctctcgcttt gccggggcga ccgacttgcg ctggatcttt 1080
gtggggagtt cgaccttgtt gctggccagc gcgcttctgt atctttgcgc ttctctgttt 1140
caaccgcgcg gaaaggtgat cgctgaaacg gtggacgtat ga 1182
<212> Type : DNA
<211> Length : 1182
   SequenceName : sequence 2
   SequenceDescription :
Feature
Sequence: sequence 2:
<221> FeatureKey : Seq ID 2 (fumA)
<222> LocationFrom : 1
<222> LocationTo : 1182
   Other Information :
   CDSJoin : No
Datebase
Sequence: sequence 2:
<308> DBAccessionNumber : FJ426269
<309> DBEntryDate : 2009-06-11
<313> From : 1
<313> To : 1182
Sequence
<213> OrganismName : Sphingopyxis sp.
<400> PresequenceString : 
MRNVSDKAPP HETLTVVVAA MIVGTAALMV LGIQPILLGA LVEEGRIPAE GLGSAATVEI 60
LAIAAGTCIG PVLMKTGYLR AKCAALCLML AAINFGLTLP GFDLPIVACR AAAGALEGLS 120
LSAAILIMTH NRRPDRLSGI FLGAQTIPQV ISAYLLPTEI IPRWGSAGGF TILGILAAIA 180
AIAALCLVDR VELDPTTVND DLQWSPAAIV ISMAAFVQFS GVGAAWSYLE RLAAQHGFSG 240
ETIGIAISGS LLCQVGGAWL AAWIGGRVGY RFALIAGSLL QAGNVIALAV ADQPSWFISA 300
SCAFGLFWLA MQPFQIRFAI AIDNSRQLAV LLTPIALVGL SAGPLLLSRF AGATDLRWIF 360
VGSSTLLLAS ALLYLCASLF QPRGKVIAET VDV 393
<212> Type : PRT
<211> Length : 393
   SequenceName : sequence 3
   SequenceDescription :
Feature
Sequence: sequence 3:
<221> FeatureKey : Seq ID 3 (FumA)
<222> LocationFrom : 1
<222> LocationTo : 393
   Other Information :
   CDSJoin : No
Datebase
Sequence: sequence 3:
<308> DBAccessionNumber : FJ426269
<309> DBEntryDate : 2009-06-11
<313> From : 1
<313> To : 393
Sequence
<213> OrganismName : Sphingopyxis sp.
<400> PreSequenceString : 
atgacatcgc aggtcaagct tcgtagcgcg gcaaagcggc cgcgcagtcc taaaagcgag 60
cgaggtcttg ctcgttacga gtccttgctt gatgcgaccg acaggctgtt ggtcgatcta 120
gaccccgatc aggtcggtct ctatcagatt gcagaggaag cgggtgcctc accgtcgtcc 180
gtctatcatt tctttccgac caaggaagtg gctcatctcg ctctgatgcg ccgctatctg 240
gaggggctcc ggaatctcga cgcgatggaa gtcgacatcg gccagctcga aagctggcag 300
gacctgatga agttggatca gatcagggcg cgagactatt ataatagcca cccgcccgcc 360
ctcaagcttc tgttcggcgg atatggcggg gtcgaggcca gaaagcttga cgagcgatac 420
tccgaggaaa tcgtgagctc catgtatggc agatacaacg gcattttcca tatgccgcaa 480
atggagaatg aggctctcat gttcacgatc tgcttcgcaa ttctcgacgc ggtatgggcc 540
gtctcctttc gccggttcgg tgaaattacg tcggatttcc ttcgggaggg gcaagcggct 600
tgcattgcct attgccgaca ctatctgccc gagcgaacgc catcagcgtg a 651
<212> Type : DNA
<211> Length : 651
   SequenceName : sequence 4
   SequenceDescription :
Feature
Sequence: sequence 4:
<221> FeatureKey : Seq ID 4 (fumB)
<222> LocationFrom : 1
<222> LocationTo : 651
   Other Information :
   CDSJoin : No
Datebase
Sequence: sequence 4:
<308> DBAccessionNumber : FJ426269
<309> DBEntryDate : 2009-06-11
<313> From : 1
<313> To : 651
Sequence
<213> OrganismName : Sphingopyxis sp.
<400> PreSequenceString : 
MTSQVKLRSA AKRPRSPKSE RGLARYESLL DATDRLLVDL DPDQVGLYQI AEEAGASPSS 60
VYHFFPTKEV AHLALMRRYL EGLRNLDAME VDIGQLESWQ DLMKLDQIRA RDYYNSHPPA 120
LKLLFGGYGG VEARKLDERY SEEIVSSMYG RYNGIFHMPQ MENEALMFTI CFAILDAVWA 180
VSFRRFGEIT SDFLREGQAA CIAYCRHYLP ERTPSA 216
<212> Type : PRT
<211> Length : 216
   SequenceName : sequence 5
   SequenceDescription :
Feature
Sequence: sequence 5:
<221> FeatureKey : Seq ID 5 (FumB)
<222> LocationFrom : 1
<222> LocationTo : 216
   Other Information :
   CDSJoin : No
Datebase
Sequence: sequence 5:
<308> DBAccessionNumber : FJ426269
<309> DBEntryDate : 2009-06-11
<313> From : 1
<313> To : 216
Sequence
<213> OrganismName : Sphingopyxis sp.
<400> PreSequenceString : 
gtggccagca agttcaactg tgagttactc gatctgcgat catttgttgc ggtgtatgaa 60
acgcgaagtt ttagccacgc cgcgcggctt ctgaatcaat cgcagcccgc gctcagccgg 120
agaatccagc gcctcgagag tctcgtgggc ggtccgttgt tcgagcggac cagtcggtcg 180
cttgccgaaa cggcgctcgg caaagagttg ctcccggtcg cccaccgagc gttggaactt 240
gtcgatacgt cgctgtttgc gtcgcccaat gtccgggagt tccgctggac agacatcacg 300
attgcctgtg tacagaccgc cgccttccat gttctcccgc gagctgcgcg cttgtacatg 360
gatcaaaatc cgagggtccg actccgcatc cttgacgtgc cggcggtcga ggctgcggac 420
ctggttgcga gcggcgaggc ggagttcggc atcagcattg agagcctgtt gccatcaagc 480
ctgcggttcg atgcgctcca cgaggacccg ttcggcctgg catgccaccg aagccatccg 540
ctggcgtcgc tcgagatcct tgaatggacg caattgaaag gtgaaagcct gatcgccgtt 600
caccgtgcga gccggaaccg cacgttgctc gatgccgaac tcgcgcgcaa caatatcgcg 660
ctggaatggc ggtatgaggt cgcgcatctg acgacggcgc tgggattgat cgatgcgcaa 720
ttgggtgtcg ctgttatgcc ccgcatggtt atgccccgct cgggtcggtc ggaggtcgtc 780
tggcgccccg tcgtcgcgcc ggtcgtccaa cgcacgatcg gcatcgttca gcgccgcacc 840
ggctcgatgc accctgccgc acagcaattg cttgcgcggc tccgcgcggc ctggtcgtcc 900
gccaatctgg gcgacatcgc gtctcgcgaa gatggggcat cgtga 945
<212> Type : DNA
<211> Length : 945
   SequenceName : sequence 6
   SequenceDescription :
Feature
Sequence: sequence 6:
<221> FeatureKey : seq ID 6 (fumC)
<222> LocationFrom : 1
<222> LocationTo : 945
   Other Information :
   COSJoin : No
Datebase
Sequence: sequence 6:
<308> DBAccessionNumber : FJ426269
<309> DBEntryDate : 2009-06-11
<313> From : 1
<313> To : 945
Sequence
<213> OrganismName : Sphingopyxis sp.
<400> PreSequenceString : 
VASKFNCELL DLRSFVAVYE TRSFSHAARL LNQSQPALSR RIQRLESLVG GPLFERTSRS 60
LAETALGKEL LPVAHRALEL VDTSLFASPN VREFRWTDIT IACVQTAAFH VLPRAARLYM 120
DQNPRVRLRI LDVPAVEAAD LVASGEAEFG ISIESLLPSS LRFDALHEDP FGLACHRSHP 180
LASLEILEWT QLKGESLIAV HRASRNRTLL DAELARNNIA LEWRYEVAHL TTALGLIDAQ 240
LGVAVMPRMV MPRSGRSEVV WRPVVAPVVQ RTIGIVQRRT GSMHPAAQQL LARLRAAWSS 300
ANLGDIASRE DGAS 314
<212> Type : PRT
<211> Length : 314 SequenceName : sequence 7 SequenceDescription :
Feature
Sequence: sequence 7:
<221> FeatureKey : Seq ID 7 (FumC)
<222> LocationFrom : 1
<222> LocationTo : 314
   Other Information :
   CDSJoin : No
Datebase
Sequence: sequence 7:
<308> DBAccessionNumber : FJ426269
<309> DBEntryDate : 2009-06-11
<313> From : 1
<313> To : 314
Sequence
<213> OrganismName : Sphingopyxis sp.
<400> PreSequenceString : 
gtgaaagagc accaatgccg tggcggccgg gcgtcccccg ctgcgcccgc cacgtggctt 60
gcgcggatca gcgtttcccg gggggcctcc gccatcgcct ggaccttcat gcttggcgca 120
actgccattc ccgtggctgc gcaaactgac gatccgaagc tcgttcgtca tacccagtcg 180
ggcgccgtcg agggcgtcga gggcgacgtc gagacttttt tgggaatacc cttcgcggct 240
ccgccggtcg gcgacctgcg atggcggccg ccggctccgc cgagggcgtg ggcgggcacc 300
agggacggcc gccgctttgc gcccgattgc atcgggaacg agcggcttag agaggggagc 360
cgggctgccg ggacgagcga agactgcctc tatctgaata tctggtctcc caaacaggtc 420
ggtaaggggg ggctccccgt catgatctgg gtttacggcg gtgggtttag cggcggttct 480
ggcgcggtgc catattatga cggctctgcg ctcgcgcaga agggcgtggt ggtcgtcacg 540
ttcaactatc gcgccgggat tctgggcttt cttgcccatc cggcgctttc aaaggaaagt 600
ccgaatggcg tgtcgggcaa ctatggtctt ctcgacatgc tcgcggcgtt caaatgggtt 660
cagaacaaca taagggagtt cggcggagac ccgaaccgtg tcacggtctt tggcgagtcc 720
gccggcgcga gcgcgctcgg actgctcctg acctcgccgc tcagtgagag cgccttcaat 780
caggcgatac tgcaaagtcc gggtctggcc aggccgctcg ccacgctttc tgaaagcgaa 840
gcgaatgggc tggagctggg agccgatatt tctgctctac ggcgtgccga tgcgggcgaa 900
ttgacgaaga tcgcgcaatc gcgaataccc atgtcgcgcc agttcaccaa gccgcggccg 960
atgggtccga ttctggacgg ctatgttttg cgcacccttg acgtcgatgc cttcgccaag 1020
 
ggggccttcc gcaagatacc cgttctggtc ggcggaaacg ccgacgaagg gcgcgctttt 1080
acggatcgcc tgccggtcaa aacggtcctt gaatatcgag cctatctcac agaacaattt 1140
ggtgacgagg cggacgcatg ggagcgttgt tatcccgcga actccgacgc cgacgtcccc 1200
gccgccgttg cccgtctttt tggggatagt cagttcaaca acgggatcga gctgctctcg 1260
gcagccttcg cgaaatggcg aacgccgctt tggagatatc gctttacggg cattccagga 1320
gccggccgtc gccccgccac gcatggagac gaaattccct atgtcttcgc aaatctgggg 1380
ccgtcgtccg tatctatgtt tgggtcgctc gaaggcggcg ccggggcgtc ggacatcaaa 1440
cttgcgaccg aaatgtccgc ggcctgggtg agcttcgcgg tgcacggggt ccccgatcag 1500
ggcacgaaat cgcactggcc gcgcttcgag cggcgagggg agatcatgac ttttggttcg 1560
caggttggct ctggggaagg tcttggagtt tcgccgagca aagcctgcca accctcaaaa 1620
tag 1623
<212> Type : DNA
<211> Length : 1623
   SequenceName : sequence 8
   SequenceDescription :
Feature
Sequence: sequence 8:
<221> FeatureKey : Seq ID 8 (fumD)
<222> LocationFrom : 1
<222> LocationTo : 1623
   Other Information :
   CDSJoin : No
Datebase
Sequence: sequence 8:
<308> DBAccessionNumber : FJ426269
<309> DBEntryDate : 2009-06-11
<313> From : 1
<313> To : 1623
Sequence
<213> OrganismName : Sphingopyxis sp.
<400> PreSequenceString : 
VKEHQCRGGR ASPAAPATWL ARISVSRGAS AIAWTFMLGA TAIPVAAQTD DPKLVRHTQS 60
GAVEGVEGDV ETFLGIPFAA PPVGDLRWRP PAPPRAWAGT RDGRRFAPDC IGNERLREGS 120
RAAGTSEDCL YLNIWSPKQV GKGGLPVMIW VYGGGFSGGS GAVPYYDGSA LAQKGVVVVT 180
FNYRAGILGF LAHPALSKES PNGVSGNYGL LDMLAAFKWV QNNIREFGGD PNRVTVFGES 240
AGASALGLLL TSPLSESAFN QAILQSPGLA RPLATLSESE ANGLELGADI SALRRADAGE 300
LTKIAQSRIP MSRQFTKPRP MGPILDGYVL RTLDVDAFAK GAFRKIPVLV GGNADEGRAF 360
TDRLPVKTVL EYRAYLTEQF GDEADAWERC YPANSDADVP AAVARLFGDS QFNNGIELLS 420
AAFAKWRTPL WRYRFTGIPG AGRRPATHGD EIPYVFANLG PSSVSMFGSL EGGAGASDIK 480
LATEMSAAWV SFAVHGVPDQ GTKSHWPRFE RRGEIMTFGS QVGSGEGLGV SPSKACQPSK 540
<212> Type : PRT
<211> Length : 540
   SequenceName : sequence 9
   SequenceDescription :
Feature
Sequence: sequence 9:
<221> FeatureKey : Seq ID 9 (FumD)
<222> LocationFrom : 1
<222> LocationTo : 540
   Other Information
   CDSJoin : No
Datebase
Sequence: sequence 9:
<308> DBAccessionNumber : FJ426269
<309> DBEntryDate : 2009-06-11
<313> From : 1
<313> To : 540
Sequence
<213> OrganismName : Sphingopyxis sp.
<400> PreSequenceString : 
ttggagtttc gccgagcaaa gcctgccaac cctcaaaata gcgcccggcc tgtgcgtgct 60
tcagcacgcc gtcccgcttt gcgggcgacg ggctgtgccc tctgcctaga aggaagtaag 120
ttgcgctacg acgtcgcgat aattggaggt ggcaacgctg cattgacggc agccgtgacg 180
gcgcgtgaag cgggggcctc ggttcttgtg atcgagcatg cgccgcgcgc catgcgcggc 240
ggcaacagtc gtcacacacg caatatgcgt acgatgcacg aacgtcccct gtcgccgttg 300
accggtgaat attcggcgga cgaatattgg aatgatcttg tccgcgtcac gggggggcgc 360
accgacgaag aactcgcgcg gctcgttatc cgcaacacca ccgacgctat tcccttcatg 420
acgcgctgcg gtgtgcgttt ccagccctcg ctgtcgggca cgctgagttt atcgcgaacc 480
aacgcattct tccttggcgg cgggaaggcg cttgtaaacg catattacgc cacggccgaa 540
cggctaggcg tcgatattct ctatgattct gaggtgaccg agatcaacct tcagcaaggc 600
gtcgtgcagc gtctgcaatt gcgcagccgg ggattccctg tcgaagtgga agccaaggct 660
gccatcgcct cgtccggagg attccaggca aatcttgact ggctctcaag cgcatggggg 720
cctgctgcgg cgaacttcat cgtacggggc acgccatatg cgactggcac ggtgctcaag 780
aacctgttgg agcaaggcgt cgcctcggtg ggagatccaa cccaatgcca tgctgtcgcg 840
atcgatgggc gagcgcccaa atacgacggc ggcatcgtca cacgactgga ctgcgttccc 900
ttctcgatcg tcgtcaacaa ggacgccttg cgcttctacg atgaaggcga agatgtgtgg 960
ccgaagcgtt acgccatatg ggtcgcttgg gtggcacagc agcctgatca gatcgctttc 1020
agcataatcg atcggcaggc cgaagacctc ttcatgccgt cagtgttccc ccccgtgcaa 1080
gcggacacga tcgcgggtct ggccgagaaa ctcggtctga atcccgtaac cctggaacgc 1140
acggtggccg aattcaacgc cgcatgcgtg cccggcgaat tcggcggcca agatctcgac 1200
gacctccaca ccgagggaat cgaaccaaag aaatccaact gggcccgacc gattattgtg 1260
cccccgttca gcgcctatcc tctccggccc gggatcacct tcacctatct cggcgtcaag 1320
gtagacagcc gtgcgcgggt catcatggag acaggtgagc cgacaaaaaa cctgtttgct 1380
tcgggggaaa taatggcggg cagcattctc ggccaaggtt atctcgctgg atttggaatg 1440
gcgattggta ccgtattcgg ccgcatcgcg ggttgggagg ccgcacgtca tgcaggattt 1500
tga 1503
<212> Type : DNA
<211> Length : 1503
   SequenceName : sequence 10
   SequenceDescription :
Feature
Sequence: sequence 10:
<221> FeatureKey : Seq ID 10 (fumE)
<222> LocationFrom : 1
<222> LocationTo : 1503
   Other Information :
   CDSJoin : No
Datebase
Sequence: sequence 10:
<308> DBAccessionNumber : FJ426269
<309> DBEntryDate : 2009-06-11
<313> From : 1
<313> To : 1503
Sequence
<213> OrganismName : Sphingopyxis sp.
<400> PreSequenceString : 
LEFRRAKPAN PQNSARPVRA SARRPALRAT GCALCLEGSK LRYDVAIIGG GNAALTAAVT 60
AREAGASVLV IEHAPRAMRG GNSRHTRNMR TMHERPLSPL TGEYSADEYW NDLVRVTGGR 120
TDEELARLVI RNTTDAIPFM TRCGVRFQPS LSGTLSLSRT NAFFLGGGKA LVNAYYATAE 180
RLGVDILYDS EVTEINLQQG VVQRLQLRSR GFPVEVEAKA AIASSGGFQA NLDWLSSAWG 240
PAAANFIVRG TPYATGTVLK NLLEQGVASV GDPTQCHAVA IDGRAPKYDG GIVTRLDCVP 300
FSIVVNKDAL RFYDEGEDVW PKRYAIWGRL VAQQPDQIAF SIIDRQAEDL FMPSVFPPVQ 360
ADTIAGLAEK LGLNPVTLER TVAEFNAACV PGEFGGQDLD DLHTEGIEPK KSNWARPIIV 420
PPFSAYPLRP GITFTYLGVK VDSRARVIME TGEPTKNLFA SGEIMAGSIL GQGYLAGFGM 480
AIGTVFGRIA GWEAARHAGF 500
<212> Type : PRT
<211> Length : 500
   SequenceName : sequence 11
   SequenceDescription :
Feature
Sequence: sequence 11:
<221> FeatureKey : Seq ID 11 (FumE)
<222> LocationFrom : 1
<222> LocationTo : 500
   Other Information :
   CDSJoin : No
Datebase
Sequence: sequence 11:
<308> DBAccessionNumber : FJ426269
<309> DBEntryDate : 2009-06-11
<313> From : 1
<313> To : 500
Sequence
<213> OrganismName : Sphingopyxis sp.
<400> PreSequenceString : 
atgcaggatt ttgatctcgt aaaaatgctg tctgacttgc cgtcggcgcc ggagctggaa 60
gccaggcgcg ttatggaggt gtgcaacgcg tgccgctatt gcgaagggtt ctgcgcggta 120
tttcctgcaa tgaccttgca gcgtcatttc gccagcggcg atctcagcca cctcgccaat 180
ctctgccact cgtgccaagg ttgctattac gcctgccaat acgcccctcc gcatgagttc 240
ggaataaacg ttccaaaggc gctgtcggag ttgcggctcg agagctacga gcagcatgct 300
tggccccggc cggtcgccgc tctctatcgc aagaatgcgc tcatcatttc catcttgtcg 360
gcggcatgca taaccggcgt ccttctgctt gccgccatct tcaacgggga tgcacttttc 420
gcgaaacacg catcggtgcc cggcggcggg ttttacaacg ttattcctta tcaggcgatg 480
attgccgtcg cggcgaccac atttctttat tccgcgctgg cgctggcgat cagtctcgtt 540
cgcttttcgc ggacgatcgg tctgggaatt aaggttcttt atcagcacgt gccggttctt 600
cgggcgctac gcgatgcggc gactctgcga tatctcggcg gcagcgacgg cgaggggtgt 660
aacgacgcgg acgagacatt ttcgacgacc cggcgaaaat ttcatcacgc ccttgcctat 720
ggcttcggac tttgtttcgc ggccacagcc acgggcacga tctacgatca tatgttcggc 780
tggccggcgc cctatgcgct tttcagcttg ccggtcgtcc tagggaccgt tgggggggatc 840
ggaatggtcg tgggcgcgat cggcctactc tggctcaagc tggccggcga agacgctcct 900
cgatcaccgg cactgcttgg gccggatgtt gccctgttgg tgcttctgct tgccatagcg 960
gcaacgggcc tcctcctttt agcggtccgc agcaccgaag tcatgggcgt cgcgctcgcc 1020
gtccatctcg gcgtcgtctt ggccttcttt ttggtgatgc catacagcaa atttgtccac 1080
ggtatcttca ggctcacggc tctcgtgcgc catcatgctg accgcgaggc aagtaatggc 1140
ttcgcctcca gccctcccac gaaaaagggt taa 1173
<212> Type : DNA
<211> Length : 1173
   SequenceName : sequence 12
   SequenceDescription :
Feature
Sequence: sequence 12:
<221> FeatureKey : Seq ID 12 (fumF)
<222> LocationFrom : 1
<222> LocationTo : 1173
   Other Information :
   CDSJoin : No
Datebase
Sequence: sequence 12:
<308> DBAccessionNumber : FJ426269
<309> DBEntryDate : 2009-06-11
<313> From : 1
<313> To : 1173
Sequence
<213> OrganismName : Sphingopyxis sp.
<400> PreSequenceString : 
MQDFDLVKML SDLPSAPELE ARRVMEVCNA CRYCEGFCAV FPAMTLQRHF ASGDLSHLAN 60
LCHSCQGCYY ACQYAPPHEF GINVPKALSE LRLESYEQHA WPRPVAALYR KNALIISILS 120
AACITGVLLL AAIFNGDALF AKHASVPGGG FYNVIPYQAM IAVAATTFLY SALALAISLV 180
RFSRTIGLGI KVLYQHVPVL RALRDAATLR YLGGSDGEGC NDADETFSTT RRKFHHALAY 240
GFGLCFAATA TGTIYDHMFG WPAPYALFSL PVVLGTVGGI GMVVGAIGLL WLKLAGEDAP 300
RSPALLGPDV ALLVLLLAIA ATGLLLLAVR STEVMGVALA VHLGVVLAFF LVMPYSKFVH 360
GIFRLTALVR HHADREASNG FASSPPTKKG 390
<212> Type : PRT
<211> Length : 390 SequenceName : sequence 13 SequenceDescription :
Feature
Sequence: sequence 13:
<221> FeatureKey : Seq ID 13 (FumF)
<222> LocationFrom : 1
<222> LocationTo : 390
   Other Information :
   CDSJoin : No
Datebase
Sequence: sequence 13:
<308> DBAccessionNumber : FJ426269
<309> DBEntryDate : 2009-06-11
<313> From : 1
<313> To : 390
Sequence
<213> OrganismName : Sphingopyxis sp.
<400> PreSequenceString : 
atggaacata tgaagtccgt tcgcgatcgc agtagcgtca tgcagatcgt gagagtggcg 60
agtggcaact gtctcgagca atatgatttc ttcgtttacg gcttctatgc ggcatatatt 120
gcgagaagct tttttccgac cggcgataac gcgacatcgc tcatgctttc attggccact 180
tttggcgctg gtttcctcat gaggcccttg gggcgatttt ttctcgggtc ctacatcgat 240
cgcgtcgggc gtcggaaagg cctgatcgtg acactcgcga tcatggccgt cggaaccctc 300
accattgcga tgactccaag ctatgaggca attggattac tcgcaccggt tatcgtgctc 360
gtcgggcgac ttttgcaggg tttttccgct ggagcagagt cgggtggcgt ctcagtgtac 420
ttggcggaaa ttgcgtcgcc caaatcgaga ggcttcttca cctcgtggca gtctgccagc 480
cagcaggtgg ccgtcatgat cgccgccgcg atcggtcttg cgctgcaatc aacgctttca 540
ccggagcaaa tgaacgactg gggatggcgg gtgcccttgt tgatcggatg cttgattatc 600
cccgtgatac tctggctgcg ccggtctctc ccggaaacga aagcctatct ccacatggag 660
cacaaggcgc attcgatcgg cgaatccctc cgcgaattgc aacagagctg gggctgatc 720
ttgacgggca tggcgatgtc gatcctcacg acgaccacct tttacatgat taccgcctat 780
acgccgacat ttggcgagaa agcactcgga ctgagcccgc aagatgtcct gctggttacc 840
atcatggtcg gcgtgtcgaa cttcctgtgg cttccgatcg gggtgctct ctcggatcgt 900
atcggtagaa ccccgatcct actggtcgtg ccggtcaccg ttctcgccat cgcctttccc 960
ctgatgagct ggctcgtcgc ggcaccgaca ttcggagcgc ttgcagctgt tctgctgact 1020
ttctccgcat gctttggact ctataatggg gcgctcatcg cgagactcac cgagattatg 1080
cctcccgcca ttagaaccct tggcttctcg ctggcgttca gtctcgcgac ctcgctgttc 1140
ggcggcttca ccccattggt aagtacggcg ctaatccacg cgacgggcag caattccgcg 1200
cctgcaatct ggctctgttt tgcggctttc atcagcttcg tcggtgtggc cgcatcgacc 1260
cggctgagcc ggccaatcgc cgaaggcgcc agatag 1296
<212> Type : DNA
<211> Length : 1296
   SequenceName : sequence 14
   SequenceDescription :
Feature
Sequence: sequence 14:
<221> FeatureKey : Seq ID 14 (fumG)
<222> LocationFrom : 1
<222> LocationTo : 1296
   Other Information :
   CDSJoin : No
Datebase
Sequence: sequence 14:
<308> DBAccessionNumber : FJ426269
<309> DBEntryDate : 2009-06-11
<313> From : 1
<313> To : 1296
Sequence
<213> OrganismName : Sphingopyxis sp.
<400> PreSequenceString : 
MEHMKSVRDR SSVMQIVRVA SGNCLEQYDF FVYGFYAAYI ARSFFPTGDN ATSLMLSLAT 60
FGAGFLMRPL GAIFLGSYID RVGRRKGLIV TLAIMAVGTL TIAMTPSYEA IGLLAPVIVL 120
VGRLLQGFSA GAESGGVSVY LAEIASPKSR GFFTSWQSAS QQVAVMIAAA IGLALQSTLS 180
PEQMNDWGWR VPLLIGCLII PVILWLRRSL PETKAYLHME HKAHSIGESL RELQQSWGLI 240
LTGMAMSILT TTTFYMITAY TPTFGEKALG LSPQDVLLVT IMVGVSNFLW LPIGGALSDR 300
IGRTPILLVV PVTVLAIAFP LMSWLVAAPT FGALAAVLLT FSACFGLYNG ALIARLTEIM 360
PPAIRTLGFS LAFSLATSLF GGFTPLVSTA LIHATGSNSA PAIWLCFAAF ISFVGVAAST 420
RLSRPIAEGA R 431
<212> Type : PRT
<211> Length : 431
   SequenceName : sequence 15
   SequenceDescription :
Feature
Sequence: sequence 15:
<221> FeatureKey : Seq ID 15 (FumG)
<222> LocationFrom : 1
<222> LocationTo : 431
   Other Information :
   CDSJoin : No
Datebase
Sequence: sequence 15:
<308> DBAccessionNumber : FJ426269
<309> DBEntryDate : 2009-06-11
<313> From : 1
<313> To : 431
Sequence
<213> OrganismName : Sphingopyxis sp.
<400> PreSequenceString : 
atgagagcag tagtttaccg aaatggcgaa cttgtcctgg gggcctatgc tgatccgata 60
cccgccgccg ggcaggtgct cgtcaagacc agagcatgcg gcatctgcgg atctgacctt 120
catttttgcg atcatgcgca ggcgtttacg aaccttgcat cgcgggcggg tatcgcctct 180
atggaagttg atttgtgtcg agacatcgtt ctggggcatg aattctgtgg cgagattatg 240
gagttcgggc cctctgcgga tcgtcgcttc aaacccggac agcttgtgtg ctcgctgccg 300
ctggcgatcg gtccgaccgg agcgcggacg attggctact cggatgagta tcccggcggg 360
ctcggcgaat atatggtcct cacggaagcg ctcttgctgc ctgttccgaa cggccttccg 420
gcgacctgcg cggcgttgac ggagccgatg gcggtgggat ggcatgccgt cgagatcgcg 480
caggttcaac cacatcacat ccctgtggtg atcgggtgcg gaccggtcgg gttggcagtc 540
gtcgctgccc tgaaacataa gcaagttgct ccgattattg cgtcggatcc atcgcccgat 600
cggcgtgctc ttgctctgcg gatgggcgcc gacgccgttg tcgatccgcg cgaagaatca 660
ccctttcgcc aggccgagaa gatcgcacgc ccggtcggac aaggtggggc cctgtccagc 720
tcattgctgt caaagtctca aatgatattc gaatgcgtag ggtgccgggg catgcttcgg 780
catgcgatgg acggcgcgtc cgacgggtcc gagatcatgg tcgttggcgc atgcatgcag 840
ccggacgcga tcgagcccat gatcgggatg tttaaagcgc tcacgatcaa attctcgcga 900
acttacacgg gtgaggaatt cgccgcggtg cttcacatga taggtgaggg cgcactcgac 960
gtatctccgc tcgttaccga tgtgattggc ctgtccgatg tcccgtccgc gtttgaggct 1020
ctacggagtc caggcgccca agcaaaagtg attgtggacc cttggcgctg a 1071
<212> Type : DNA
<211> Length : 1071
   SequenceName : sequence 16
   SequenceDescription :
Feature
Sequence: sequence 16:
<221> FeatureKey : Seq ID 16 (fumH) <222> LocationFrom : 1
<222> LocationTo : 1071
   Other Information :
   CDSJoin : No
Datebase
Sequence: sequence 16:
<308> DBAccessionNumber : FJ426269
<309> DBEntryDate : 2009-06-11
<313> From : 1
<313> To : 1071
Sequence
<213> OrganismName : Sphingopyxis sp.
<400> PreSequenceString : 
MRAVVYRNGE LVLGAYADPI PAAGQVLVKT RACGICGSDL HFCDHAQAFT NLASRAGIAS 60
MEVDLCRDIV LGHEFCGEIM EFGPSADRRF KPGQLVCSLP LAIGPTGART IGYSDEYPGG 120
LGEYMVLTEA LLLPVPNGLP ATCAALTEPM AVGWHAVEIA QVQPHHIPVV IGCGPVGLAV 180
VAALKHKQVA PIIASDPSPD RRALALRMGA DAVVDPREES PFRQAEKIAR PVGQGGALSS 240
SLLSKSQMIF ECVGVPGMLR HAMDGASDGS EIMVVGACMQ PDAIEPMIGM FKALTIKFSR 300
TYTGEEFAAV LHMIGEGALD VSPLVTDVIG LSDVPSAFEA LRSPGAQAKV IVDPWR 356
<212> Type : PRT
<211> Length : 356
   SequenceName : sequence 17
   SequenceDescription :
Feature
Sequence: sequence 17:
<221> FeatureKey : Seq ID 17 (FumH)
<222> LocationFrom : 1
<222> LocationTo : 356
   Other Information :
   CDSJoin : No
Datebase
Sequence: sequence 17:
<308> DBAccessionNumber : FJ426269 <309> DBEntryDate : 2009-06-11
<313> From : 1
<313> To : 356
Sequence
<213> OrganismName : Sphingopyxis sp.
<400> PreSequenceString : 
atggcgaacg gaacaaggca gaaagatctc agagaacgcg ccgaacgggt cattccgggc 60
gggatgtacg gccacgagtc gacacggttg ctgccgccag aattccccca gttcttcagg 120
cgcgcgctgg gggcacgaat ttgggacgcc gacgagcagc cctatatcga ctatatgtgc 180
gcgtatgggc caaatttgct cggttaccgg caatccgaaa tcgaagccgc ggctgatgcg 240
cagcgacttc tcggcgacac catgaccggt ccttcggaga tcatggtcaa cctcgccgaa 300
gcctttgtgg gcatggtccg tcatgcggat tgggcgatgt tctgcaaaaa tggcagcgat 360
gccacctcaa cggcgatggt tctcgcgcgt gcccatacgg ggcgcaaaac catattatgc 420
gccaaaggcg cctatcatgg cgcttccccg tggaacactc cgcatactgc cgggattctc 480
gcttccgatc gcgtgcatgt cgcatattat acctataacg acgcccaaag cttatcggac 540
 
gcgttcaagg cgcacgatgg cgatattgcg gctgtctttg ccacaccttt ccgacacgaa 600
gtatttgagg accaggccct cgcccagctt gagttcgcgc gcaccgctcg aaaatgttgt 660
gacgagaccg gtgcgcttct ggtcgttgac gatgtgcgcg caggtttccg ggtggcgcgc 720
gattgcagct ggacgcattt gggtatcgaa cccgatctca gttgctgggg aaaatgcttt 780
gcgaatggct atccgatctc cgccctgctg ggctcgaaca aggcgcgcga tgcggcgcgg 840
gatatatttg tgaccggctc cttctggttc tctgcggtac cgatggcggc cgcgatcgaa 900
accctcagga tcattcgaga gacgccttat ctcgaaacgc tgatcgccag cggcgccgcc 960
ctgcgggcag gcctggaggc acagtctcag cgccatggtc ttgagttgaa gcagacgggc 1020
ccggcgcaga tgccgcaaat attctttgcg gacgatcccg attttcggat cggctatgcg 1080
tgggccgcgg cgtgcctgaa gggcggcgtc tatgttcatc cctatcacaa tatgtttctc 1140
tctgcggccc atacagttga cgatgtaacg gagaccctcg aggcgacgga tcgcgcgttc 1200
agcgcggtcc tcagagattt tgcgtctctc cagcctcatc ccattttaat gcaactcgcc 1260
ggtgcttga 1269
<212> Type : DNA
<211> Length : 1269
   SequenceName : sequence 18
   SequenceDescription :
Feature
Sequence: sequence 18:
<221> FeatureKey : Seq ID 18 (fumI)
<222> LocationFrom : 1
<222> LocationTo : 1269
   Other Information :
   CDSJoin : No
Datebase
Sequence: sequence 18:
<308> DBAccessionNumber : FJ426269 <309> DBEntryDate : 2009-06-11
<313> From : 1
<313> To : 1269
Sequence
<213> OrganismName : Sphingopyxis sp.
<400> PreSequenceString : 
MANGTRQKDL RERAERVIPG GMYGHESTRL LPPEFPQFFR RALGARIWDA DEQPYIDYMC 60
AYGPNLLGYR QSEIEAAADA QRLLGDTMTG PSEIMVNLAE AFVGMVRHAD WAMFCKNGSD 120
ATSTAMVLAR AHTGRKTILC AKGAYHGASP WNTPHTAGIL ASDRVHVAYY TYNDAQSLSD 180
AFKAHDGDIA AVFATPFRHE VFEDQALAQL EFARTARKCC DETGALLVVD DVRAGFRVAR 240
DCSWTHLGIE PDLSCWGKCF ANGYPISALL GSNKARDAAR DIFVTGSFWF SAVPMAAAIE 300
TLRIIRETPY LETLIASGAA LRAGLEAQSQ RHGLELKQTG PAQMPQIFFA DDPDFRIGYA 360
WAAACLKGGV YVHPYHNMFL SAAHTVDDVT ETLEATDRAF SAVLRDFASL QPHPILMQLA 420
GA 422
<212> Type : PRT
<211> Length : 422
   SequenceName : sequence 19
   SequenceDescription :
Feature
Sequence: sequence 19:
<221> FeatureKey : Seq ID 19 (FumI)
<222> LocationFrom : 1
<222> LocationTo : 422
   Other Information :
   CDSJoin : No
Datebase
Sequence: sequence 19:
<308> DBAccessionNumber : FJ426269
<309> DBEntryDate : 2009-06-11
<313> From : 1 <313> To : 422
Sequence
<213> OrganismName : Sphingopyxis sp.
<400> PreSequenceString : 
atgtatcgga agttcagaat cgaaaagccc ggcaaggcaa atagtttgct cggcgcagta 60
gcgctcggca ccctcgcatt tcctgtctct gccagtgctc aggatagcga tcccgcatcg 120
ataggtcagc cggacgaagc ggacacggac cggggaacga gcgaaatcgt cgtgaccggc 180
agccgcctcc agaacggctt caattcgccg acgccggtta cagccgtatc cagcgagcag 240
ttgaaggagg catctccgac caaccttgcc gacgcactca accagctgcc cgtgttcaac 300
gacagcttga agacctccaa ccctggcacg acacccggaa cggggaacag cggtcagaac 360
ctgctcaaca tgcgcggcct cgggtcaaac cggaacctcg tcctgctgaa cggcaaccgt 420
ttcgtcgcga ccaatttcac aggctcggtc gatatcaacg tgctgccgca ggcgttggtc 480
aagcgcgtcg atgtcgtgac gggcggcgcc tcggccgcct acggttccga tgccgtttcg 540
ggcgtcatca acttcgtgct cgacgaagat ctggaaggca tcagggccga gctccagtcg 600
ggtgtttcaa cccgcggcga cctcccgtcc tacggcggtt cgatcgcctt cggcacttcg 660
tttgccgacg accggttgca cttgctcggc agcttcgaat attttcgaca ggacggaatc 720
cgggccgatg aagcaacggg tcgccgctgg ttcgacatcg ccgccggcca atatcccgtg 780
cccggcgcta cgacaggcgt cacggtcgtg cccgatattc gcagttctcg cggatcctac 840
ggcggacttg tcacgtccgg ccctctgaaa ggcatcgcgt ttttgcccgg aggagtccta 900
gggaccttcg actacgggaa ttttacgagc tcgtcgttcc agagcggcgg cgatggaccg 960
cgcgtgaata tcggcttcgc cccggatcag cttcgctaca acgcgttcct acgcgccgca 1020
tatgatgtgt ccgacactgt gcaggtgtat gcggagggca cctatgctta ttcccacacc 1080
aacctgggtg cattcgtaat atcgcatgtc ggtggctcga ataatttccg gatcttccgt 1140
gataacgcct tccttccggc tccactcgcg acgctcatgg acagaaatgc ccaggcttcg 1200
atcgttgtcg gtcgcttctc aagcgacttt cccttggtcg aaatcgagaa tttcgcaaag 1260
gtctaccgcg gcgctgccgg cttccgggca gacattggca atggctggaa actcgatggc 1320
tcggcctcct ttggccttac ggacctcgag cttcgtgaaa acaatctcac catcaaccgc 1380
aatctctacg ccgccgtcga tgcggtccgc gatcccgcgg gcaatatcgt ctgccgttca 1440
acactggccg gcctcgacca agattgcgtg ccgctcaatc tcttcggcac aggctcgccg 1500
agcgcgtcgg ccatcgacta tgtcaccgct gatggcgtcg ctcagctgag gcttgagcaa 1560
tatgtggcgg gactcacgat ttccggcgac ctcggcgata gcctgtcgtt cggcgcgggc 1620
ccggtctcgg tcgccgctgg tatcgaatat cgcaaggaga aggcccggca ggaaaccgac 1680
gcgatatcgc aggcgacgac ctcgatcacg ggaatcaggg gggctccggc ggcgcaggca 1740
ggtcggcctg gaggcttcaa tctctacaac ccacttccct tctcgggaag ctatgacatc 1800
aaggaaggtt ttgtcgaaat cggcgtcccg attctgaagg acagcgcgct gggacgttcg 1860
ctgaacttaa acggcgccgt ccgatatgcc gattacagcc agtccggtgg agtaacaacc 1920
tggaagctgg gcggagaata tgagccgatc gacggcctca ggttccgcgc gacccgttcg 1980
cgagatatcc gcgggccaag ccttgtcgag ctattcgacc ccggccgtca ggcgacgctc 2040
aattcaattt atggcggaca ggctgtgcag acgcggttct ttaccgccgg caacgcggat 2100
ttgcgccctg aaaaggcgga cgtccttaca ttcggcgcgg tgctacgccc cgccttcgtg 2160
ccggggtttc agttttcggt cgatcgctat gtggtgaagg tgaagggcgc gatcgatttc 2220
ctccttcccc agcaggaaat cgacgcgtgc gatgcaggaa acaccttctt ctgcgacctc 2280
ataacggaga atccggacgg caccatcaca gtgacgggtc ccaatctcaa cctggctgtc 2340
cagaaagcgg cgggaattga cttcgaggcc tattactcac gccccgtcgg cggcggcacg 2400
ttcagtcttc gtgcgctggc aacgcaccat acctctgcct atcgcatcgc gaccggctcg 2460
gcgcccatcc gttcgctcgg acaaccggac acgccaaaat ggtcggccaa cttccaggcg 2520
cgatattcga ccgacgattg ggcgcttctc gtgcagcagc gcttcatcgc agcatcggtg 2580
ttcaatgccg acaatgtgga gggcgtcgat acgaatttga accacgctcc ggcggtttgg 2640
tacaccgacg cgacattgac cttcgacatc gcggcttttg gccagaagca gcagctgttt 2700
ctatcggtca ataatttgtt cgaccgagat ccgccaatag cgacgaacga ccccagcagt 2760
ttttccagcc cgaccagctc tgcctatgat ccggtcggcc gctattttaa tgtcggggtc 2820
cgtttccgga tctga 2835
<212> Type : DNA
<211> Length : 2835
   SequenceName : sequence 20
   SequenceDescription :
Feature
Sequence: sequence 20:
<221> FeatureKey : Seq ID 20 (fumJ)
<222> LocationFrom : 1
<222> LocationTo : 2835
   Other Information
   CDSJoin : No
Datebase
Sequence: sequence 20:
<308> DBAccessionNumber : FJ426269
<309> DBEntryDate : 2009-06-11
<313> From : 1
<313> To : 2835
Sequence
<213> OrganismName : Sphingopyxis sp.
<400> PreSequenceString : 
MYRKFRIEKP GKANSLLGAV ALGTLAFPVS ASAQDSDPAS IGQPDEADTD RGTSEIVVTG 60
SRLQNGFNSP TPVTAVSSEQ LKEASPTNLA DALNQLPVFN DSLKTSNPGT TPGTGNSGQN 120
LLNMRGLGSN RNLVLLNGNR FVATNFTGSV DINVLPQALV KRVDVVTGGA SAAYGSDAVS 180
GVINFVLDED LEGIRAELQS GVSTRGDLPS YGGSIAFGTS FADDRLHLLG SFEYFRQDGI 240
RADEATGRRW FDIAAGQYPV PGATTGVTVV PDIRSSRGSY GGLVTSGPLK GIAFLPGGVL 300
GTFDYGNFTS SSFQSGGDGP RVNIGFAPDQ LRYNAFLRAA YDVSDTVQVY AEGTYAYSHT 360
NLGAFVISHV GGSNNFRIFR DNAFLPAPLA TLMDRNAQAS IVVGRFSSDF PLVEIENFAK 420
VYRGAAGFRA DIGNGWKLDG SASFGLTDLE LRENNLTINR NLYAAVDAVR DPAGNIVCRS 480
TLAGLDQDCV PLNLFGTGSP SASAIDYVTA DGVAQLRLEQ YVAGLTISGD LGDSLSFGAG 540
PVSVAAGIEY RKEKARQETD AISQATTSIT GIRGAPAAQA GRPGGFNLYN PLPFSGSYDI 600
KEGFVEIGVP ILKDSALGRS LNLNGAVRYA DYSQSGGVTT WKLGGEYEPI DGLRFRATRS 660
RDIRGPSLVE LFDPGRQATL NSIYGGQAVQ TRFFTAGNAD LRPEKADVLT FGAVLRPAFV 720
PGFQFSVDRY VVKVKGAIDF LLPQQEIDAC DAGNTFFCDL ITENPDGTIT VTGPNLNLAV 780
QKAAGIDFEA YYSRPVGGGT FSLRALATHH TSAYRIATGS APIRSLGQPD TPKWSANFQA 840
RYSTDDWALL VQQRFIAASV FNADNVEGVD TNLNHAPAVW YTDATLTFDI AAFGQKQQLF 900
LSVNNLFDRD PPIATNDPSS FSSPTSSAYD PVGRYFNVGV RFRI 944
<212> Type : PRT
<211> Length : 944
   SequenceName : sequence 21
   SequenceDescription :
Feature
Sequence: sequence 21:
<221> FeatureKey : Seq ID 21 (FumJ)
<222> LocationFrom : 1
<222> LocationTo : 944
   Other Information :
   CDSJoin : No
Datebase
Sequence: sequence 21:
<308> DBAccessionNumber : FJ426269
<309> DBEntryDate : 2009-06-11
<313> From : 1
<313> To : 944
Sequence
<213> OrganismName : Sphingopyxis sp.
<400> PreSequenceString : 
atgcgcctca cgggcggaga attattggca cgatgtttgg ccgtcgaagg cgtccggtat 60
gtcttcggcc tcatgtcgcc ggaggtggat ccgctcctgg ctgcgctcga agacaatggg 120
atattgttcg tcccggtgcg gcacgaggcc gccgcagcct atatggccga gggcatttac 180
aagaccaccg gacaggtcgc cgcgattgtc acgaatccgg gtcccggtac ggcaaacctt 240
ctgcctggag tcgtgacggc acgccacgaa ggggttccct tcgtcgcaat aacgtcccag 300
catcaacttg gtgtcgttta tccctgcacg ccaaaaacct ttcaggacaa agaccagatc 360
gacctctttc gacccgcggt taaatggggc gcacccatct tcgcctggaa ccggatt 417
<212> Type : DNA
<211> Length : 417
   SequenceName : sequence 22
   SequenceDescription :
Feature
Sequence: sequence 22:
<221> FeatureKey : Seq ID 22 (fumK)
<222> LocationFrom : 1
<222> LocationTo : 417
   Other Information :
   CDsJoin : No
Datebase
Sequence: sequence 22:
<308> DBAccessionNumber : FJ426269
<309> DBEntryDate : 2009-06-11
<313> From : 1
<313> To : 417
Sequence
<213> OrganismName : Sphingopyxis sp.
<400> PreSequenceString : 
MRLTGGELLA RCLAVEGVRY VFGLMSPEVD PLLAALEDNG ILFVPVRHEA AAAYMAEGIY 60
KTTGQVAAIV TNPGPGTANL LPGVVTARHE GVPFVAITSQ HQLGVVYPCT PKTFQGQDQI 120
DLFRPAVKWG APIFAWNRI 139
<212> Type : PRT
<211> Length : 139
   SequenceName : sequence 23
   sequenceDescription :
Feature
Sequence: sequence 23:
<221> FeatureKey : Seq ID 23 (FumK)
<222> LocationFrom : 1
<222> LocationTo : 139
   Other Information :
   CDSJoin : No
Datebase
Sequence: sequence 23:
<308> DBAccessionNumber : FJ426269
<309> DBEntryDate : 2009-06-11
<313> From : 1
<313> To : 139
Sequence
<213> OrganismName : Caulobacter sp.
<400> PreSequenceString : 
atggaattga gccgccaacg agaccaggcc ttgagggagc gcgcccaagc ggtgatcccg 60
ggcgggatgt acggtcacga gtcgacctat ctgatgcccg agggcacgcc acagttcttc 120
agtcgcggca aaggcgcccg actttgggac gccgacggca acgagtatgt cgattacatg 180
tgcgcctatg gccccaacct gctgggttac ggcttcgaac ccgtcgaagc ggccgccgca 240
gcccagcaag cccggggcga taccctgacc gggccgtcgg aggtgatggt gcagttggcg 300
gaagacttcg tcgcgcaaat cagccacgcg gactgggcca tgttctgcaa gaacggcaca 360
gacgccacct caatggcgat ggtcatcgcg cgcgcacaca ccggccggaa gacgatcctc 420
tgcgcgaaag gcgcctatca tggggccgcg ccttggtgca cgccgatcct ggccggaacg 480
ctaccggagg atcgcgcctt tgtagtctac tacgactaca atgacgccca aagcctcgtc 540
gacgccttcg aggcccatca ggacgacgtc gcggcgatct tcgccacccc tcaccgtcac 600
gaggtgttca gcgaccagat cgatcctgat ccggaatatg cggccagcgt gcgggcgctc 660
tgcgacaaga gcggcgccct gctcgtcgtc gacgaagttc gagccgggtt caggatcgcg 720
cgcgactgca gctgggccaa gatcggcgtc gctccggatc tgagcacctg gggcaagtgc 780
ttcgccaacg gctatccgat ctcggcggtc ctagggggcg aaaaggtgcg cagcgcggca 840
aaggccgtct acgtcaccgg ctcgttctgg ttctcggcca cgcccatggc cgcagccgtc 900
gaaaccctga agcaaatccg cgagaccgac tatctcgagc ggatcaacgc ggccgggacc 960
cgcctgcgcg agggcctgca gcagcaggct gctcacaacg gctttacgtt gcgccaaacg 1020
gggcccgtct ccatgcccca agtcctcttc gaggaagatc ccgattttcg ggtcggctac 1080
ggctgggttc gcgaatgcct gaagcgaggg gtgtacttca gcccctacca taacatgttc 1140
ctgtcggcgg cccatagcga ggcggacctg gccaagaccc ttgcggctac cggcgacgcc 1200
 
ttcgtcgagc tacgcgccaa gcttccgagc ctagaaatcc accaacccct cctcgccctg 1260
agagcggcct aa 1272
<212> Type : DNA
<211> Length : 1272
   SequenceName : sequence 24
   SequenceDescription :
Feature
Sequence: sequence 24:
<221> FeatureKey : Seq ID 24
<222> LocationFrom : 1
<222> LocationTo : 1272
   Other Information :
   CDSJoin : No
Datebase
Sequence: sequence 24:
<308> DBAccessionNumber : FJ426269
<309> DBEntryDate : 2009-06-11
<313> From : 1
<313> To : 1272
Sequence
<213> OrganismName : Caulobacter sp.
<400> PreSequenceString : 
MELSRQRDQA LRERAQAVIP GGMYGHESTY LMPEGTPQFF SRGKGARLWD ADGNEYVDYM 60
CAYGPNLLGY GFEPVEAAAA AQQARGDTLT GPSEVMVQLA EDFVAQISHA DWAMFCKNGT 120
DATSMAMVIA RAHTGRKTIL CAKGAYHGAA PWCTPILAGT LPEDRAFVVY YDYNDAQSLV 180
DAFEAHQDDV AAIFATPHRH EVFSDQIDPD PEYAASVRAL CDKSGALLVV DEVRAGFRIA 240
RDCSWAKIGV APDLSTWGKC FANGYPISAV LGGEKVRSAA KAVYVTGSFW FSATPMAAAV 300
ETLKQIRETD YLERINAAGT RLREGLQQQA AHNGFTLRQT GPVSMPQVLF EEDPDFRVGY 360
GWVRECLKRG VYFSPYHNMF LSAAHSEADL AKTLAATGDA FVELRAKLPS LEIHQPLLAL 420
RAA 423
<212> Type : PRT
<211> Length : 423
   SequenceName : sequence 25
   SequenceDescription :
Feature
sequence: sequence 25:
<221> FeatureKey : Seq ID 25
<222> LocationFrom : 1
<222> LocationTo : 423
   Other Information :
   CDSJoin : No
Datebase
Sequence: sequence 25:
<308> DBAccessionNumber : FJ426269
<309> DBEntryDate : 2009-06-11
<313> From : 1
<313> To : 423

## Patentansprüche

1. Futtermittelzusatz geeignet zum sauerstoffunabhängigen, enzymatischen Abbau von Fumonisinen, in Futtermitteln bzw. im Verdauungstrakt von Tieren, **dadurch gekennzeichnet, dass** ein Enzym der Sequenz ID-Nr. 9 sowie gegebenenfalls zusätzlich ein Enzym der Sequenz ID-Nr. 19, ein Cosubstrat für das eingesetzte Enzym und ein inerter Träger enthalten sind.

2. Futtermittelzusatz nach Anspruch 1, **dadurch gekennzeichnet, dass** das Enzym in einer Schutzhülle verkapselt einigesetzt ist.

3. Futtermittelzusatz nach Anspruch 1 oder 2, **dadurch gekennzeichnet, dass** eine Carboxylesterase der Sequenz ID-Nr. 9, eine Aminotransferase der Sequenz ID-Nr. 19, eine α-Ketosäure als Cosubstrat und ein inerter Träger enthalten sind.

4. Verwendung eines Gens mit der Sequenz ID-Nr. 8 zur Expression einer Enzymsequenz der Sequenz ID-Nr. 9.

5. Verwendung des Enzyms der Sequenz ID-Nr. 9 sowie gegebenenfalls des Enzyms der Sequenz ID-Nr. 19 und eines Cosubstrates zur Herstellung eines Futtermittelzusatzes für Tiere.

6. Verwendung nach Anspruch 5, **dadurch gekennzeichnet, dass** das Cosubstrat eine α-Ketosäure ist und ein inerter Träger eingesetzt wird.

## Claims

1. An animal feed additive for the oxygen-independent enzymatic degradation of fumonisins in animal feed or in the digestive tract of animals, **characterized in that** it contains an enzyme of the sequence ID No. 9 as well as optionally one, in addition, one enzyme of the sequence ID No. 19, or a cosubstrate for the enzyme used, and an inert carrier.

2. An animal feed additive according to claim 1, **characterized in that** the enzyme is used encapsulated in a protective cover.

3. An animal feed additive according to claim 1 or 2, **characterized in that** a carboxylesterase with sequence ID No. 9, an aminotransferase with sequence ID No. 19, an α-keto acid and an inert carrier are contained.

4. The use of a gene of the sequence ID No. 8 for expressing an enzymatic sequence of the sequence ID No. 9.

5. The use of an enzyme of the sequence ID No. 9 as well as optionally one enzyme of the sequence ID No. 19, and a cosubstrate for the production of a feed additive for animals.

6. The use according to claim 5, **characterized in that** the cosubstrate is an α-keto acid and an inert carrier is used.

## Revendications

1. Additif pour l'alimentation animale convenant à la dégradation anaérobique enzymatique de fumonisines dans des aliments pour animaux ou dans le tube digestif d'animaux, **caractérisé en ce qu'**une enzyme de la séquence ID n° 9 ainsi que le cas échéant en supplément une enzyme de la séquence ID n° 19, un cosubstrat pour l'enzyme utilisée et un support inerte sont contenus.

2. Additif pour l'alimentation animale selon la revendication 1, **caractérisé en ce que** l'enzyme est utilisée sous une forme encapsulée dans une enveloppe de protection.

3. Additif pour l'alimentation animale selon la revendication 1 ou 2, **caractérisé en ce qu'**une carboxylestérase de la séquence ID n° 9, une aminotransférase de la séquence ID n° 19, un α-acide cétonique en tant que cosubstrat et un support inerte sont contenus.

4. Utilisation d'un gène avec la séquence ID n° 8 servant à l'expression d'une séquence enzymatique de la séquence ID n° 9.

5. Utilisation de l'enzyme de la séquence ID n° 9 ainsi que le cas échéant de l'enzyme de la séquence ID n° 19 et d'un co-substrat pour la fabrication d'un additif pour l'alimentation des animaux.

6. Utilisation selon la revendication 5, **caractérisée en ce que** le co-substrat est un α-acide cétonique, et **en ce qu'**un support inerte est utilisé.
